(19) Europäisches Patentamt
European Patent Office
Office européen des brevets

(11) Publication number: 0 137 242 B1

# (12) EUROPEAN PATENT SPECIFICATION

(45) Date of publication of patent specification: 13.05.92

(51) Int. Cl.[5]: C07D 413/12, A61K 31/42, C07D 263/14

(21) Application number: 84109911.2

(22) Date of filing: 20.08.84

## (54) (Substituted) Phenyl-aliphatic-isoxazoles useful as antiviral agents and preparation thereof.

(30) Priority: 29.08.83 US 527583
25.06.84 US 624302

(43) Date of publication of application:
17.04.85 Bulletin 85/16

(45) Publication of the grant of the patent:
13.05.92 Bulletin 92/20

(84) Designated Contracting States:
AT BE CH DE FR GB IT LI NL SE

(56) References cited:
EP-A- 0 111 345
US-A- 4 268 678

(73) Proprietor: STERLING DRUG INC.
90 Park Avenue
New York New York(US)

(72) Inventor: Guy, Dominic Diana
Box 192 Garfield Road
East Nassau, NY(US)

(74) Representative: Baillie, Iain Cameron et al
c/o Ladas & Parry, Altheimer Eck 2
W-8000 München 2(DE)

EP 0 137 242 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to novel (substituted) phenyl-aliphatic isoxazoles useful as antiviral agents and, to the preparation thereof.

U.S. Patent 4,268,678, discloses antivirally active compounds having the formula:

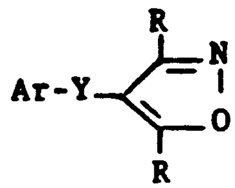

wherein Ar is phenyl substituted by one or two substituents selected from the group consisting of halogen, lower-alkoxy, nitro and hydroxy; Y is $(CH_2)_n$ or $O(CH_2)_n$ where n is an integer from 1 to 8; and R is lower-alkyl.

The present invention relates to compounds having the formulas

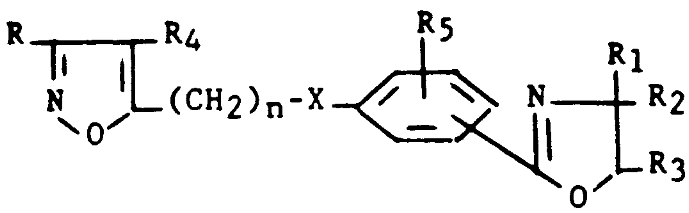

I

or

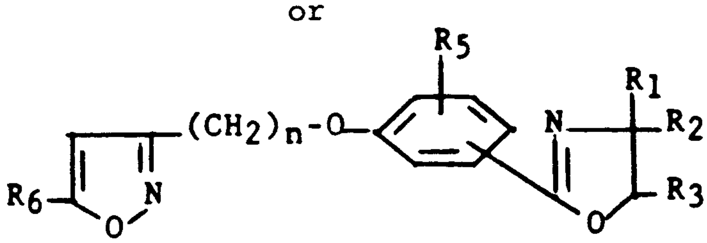

II

wherein:

R, $R_1$, $R_2$, $R_3$ and $R_4$ are each hydrogen or alkyl of 1 to 3 carbon atoms optionally substituted by hydroxy, lower-alkanoyloxy of from 1 to 4 carbon atoms, lower-alkoxy of from 1 to 4 carbon atoms, chloro, or N=Z, wherein N=Z is amino, lower alkanoylamino of from 1 to 4 carbon atoms, 1-pyrrolidinyl, 1-piperidinyl or 4-morpholinyl; with the proviso that R is other than hydrogen;

$R_5$ is hydrogen, lower-alkyl of 1-4 carbon atoms, halogen, nitro, lower-alkoxy of 1-4 carbon atoms, lower-alkylthio of 1-4 carbon atoms or trifluoromethyl;

$R_6$ is alkyl of 1 to 3 carbon atoms;

X is O or a single bond; and

n is an integer from 3 to 9;

and to pharmaceutically acceptable acid-addition salts thereof.

The invention further relates to compounds having the formulas

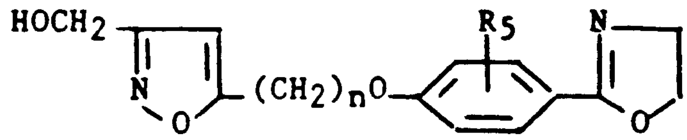

III

and

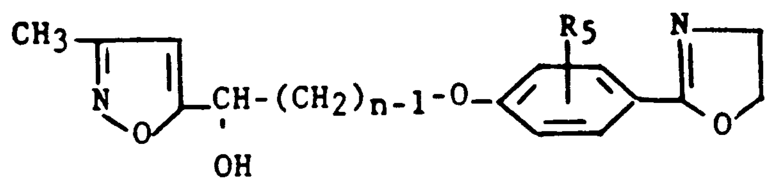

IV

wherein $R_5$ and n have the meanings given above, which are microbial conversion products of compounds of Formula I where X = 0, R = $CH_3$, $R_1$, $R_2$, $R_3$ and $R_4$ = H, and the dihydro-oxazole moiety is in the 4-position in the phenylene ring. It is noted that compounds of the Formula III fall within the scope of Formula I.

Compositions for combatting viruses comprise an antivirally effective amount of a compound of Formulas I, II, III or IV in admixture with a suitable carrier or diluent.

A process for preparing a compound within the scope of Formula I or II having the formulas

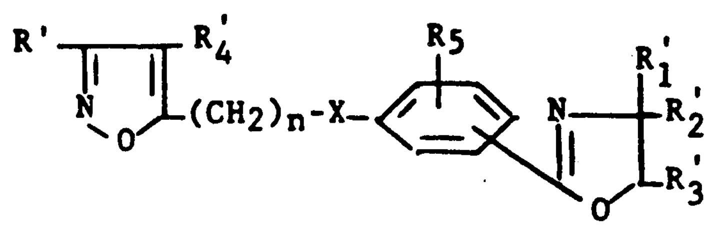

or

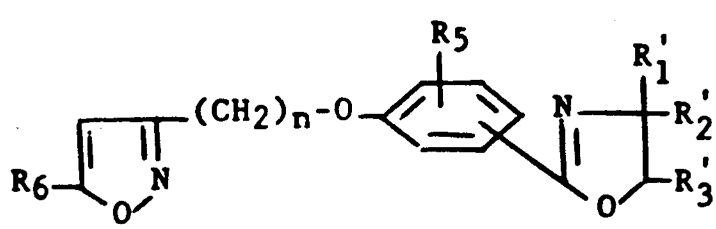

wherein R' is alkyl or hydroxyalkyl of 1 to 3 carbon atoms; $R'_1$ , $R'_2$ , $R'_3$ and $R'_4$ are each hydrogen, or alkyl or hydroxyalkyl of 1 to 3 carbon atoms; and $R_5$, $R_6$, X and n have the meanings given above, comprises reacting a compound of the formula

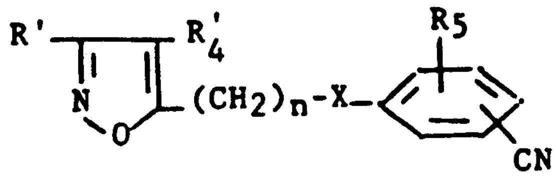

V

or

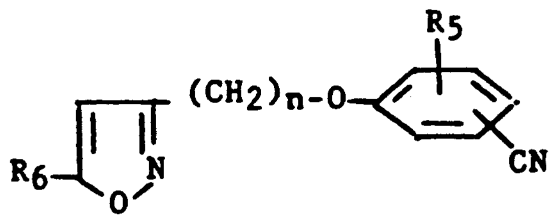

VI

with a lower-alkanol in the presence of a strong acid, and heating the resulting imino ester with a compound of the formula

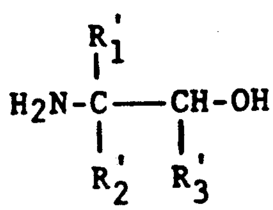

A process for preparing a compound within the scope of Formula I where X is O having the formula

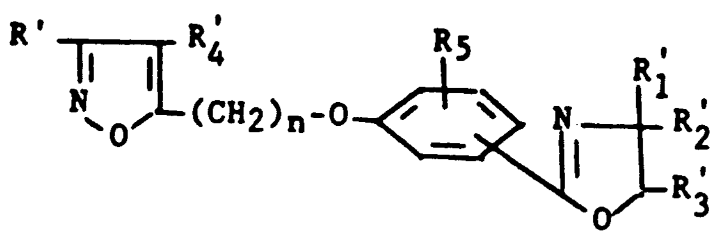

wherein R' is alkyl or hydroxyalkyl of 1 to 3 carbon atoms; $R'_1$ , $R'_2$ , $R'_3$ and $R'_4$ are each hydrogen, or alkyl or hydroxyalkyl of 1 to 3 carbon atoms; and $R_5$ and n have the meanings given above, comprises:

(a) reacting a compound of the formula

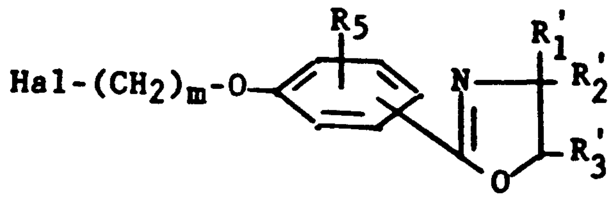

VII

wherein Hal is bromine or iodine, and m = n-1, with an alkali metal derivative of 3-R'-4-$R_4'$ -5-methylisoxazole; or

(b) reacting a compound of the formula

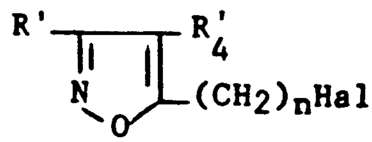

VIII

with an alkali metal salt of a compound of the formula

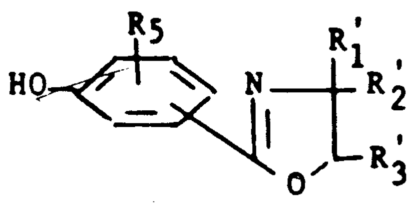

IX .

compounds of formula IX wherein the hydroxy and oxazole groups are in a position <u>para</u> to each other, provided that not all $R_1'$ , $R_2'$ , $R_3'$ and $R_5$ are hydrogen, are believed to be novel.

A method for combatting viruses comprises contacting the locus of said viruses with a composition containing an antivirally effective amount of a compound of Formula I, II, III or IV.

The invention further relates to intermediates of the formulas VII and IX above.

## DETAILED DESCRIPTION INCLUSIVE OF PREFERRED EMBODIMENTS

The compounds of Formulas I-IV are basic enough to form stable acid-addition salts with strong acids, and said salts are within the purview of the invention. The nature of the acid-addition salt is immaterial provided it is derived from an acid the anion of which is essentially non-toxic to animal organisms. Examples of appropriate acid-addition salts include the hydrochloride, hydrobromide, sulfate, acid sulfate, maleate, citrate, tartrate, methanesulfonate, p-toluenesulfonate, cyclohexanesulfamate, and the like.

In the compounds of the invention wherein $R_5$ is lower-alkyl, lower-alkoxy or lower-alkylthio, the alkyl moiety preferably has from one to four carbon atoms; and in compounds where $R_5$ is halogen, any of the four common halogens, fluorine, chlorine, bromine, or iodine are contemplated with fluorine and chlorine being preferred.

In the process involving the conversion of the intermediates of Formulas V and VI to the final products of Formulas I and II, respectively, the first step, conversion of the nitrile to an imino ester:

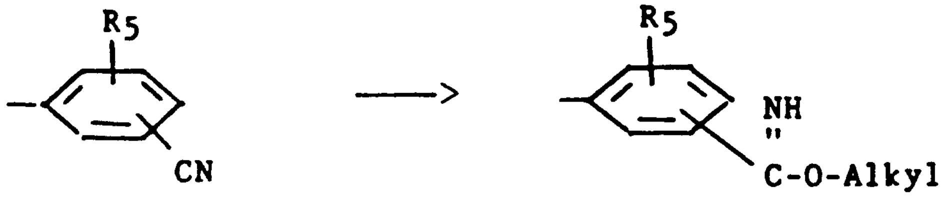

is carried out by treating the nitrile with a lower-alkanol, preferably methanol or ethanol in the presence of a strong acid. A preferred procedure is to saturate a solution of the nitrile in a lower-alkanol with gaseous hydrogen chloride at a reduced temperature (0° to -70°C) and allow the mixture gradually to warm to room temperature until the reaction is complete. The hydrochloride salt of the imino ester is obtained.

The second step is the reaction of the imino ester with a hydroxyalkylamine of the formula $H_2NC(R_1' R_2')CH(R_3')OH$. The reaction can be carried out by heating the reactants together at a temperature between about 100°C and 150°C.

The intermediate nitriles of Formula V are in turn prepared by reacting the alkali metal derivative of a 3-R-5-methylisoxazole with a halide of the formula:

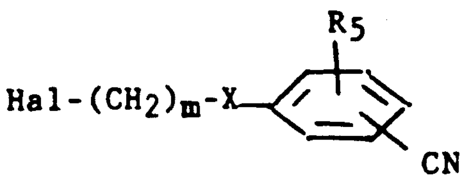

X

where Hal is bromine or iodine and m = n-1.

The compounds of Formula X where X is O are obtained by reacting cyanophenol with a dihalide of the formula $Hal-(CH_2)_m-Hal$ in the presence of a base.

The compounds of Formula X where X is a single bond are prepared by procedures analogous to those shown in U.S. Pat. 4,093,736 (June 6, 1978) starting with the appropriate cyanophenyl compounds. For example, starting with 4-cyanobenzaldehyde and methyl cyclopropyl ketone, and following the reaction of sequence A of said patent, there is obtained 6-(4-cyanophenyl)hexyl bromide.

The intermediate nitriles of Formula VI are prepared by reacting a cyanophenol under basic conditions with a compound of the formula

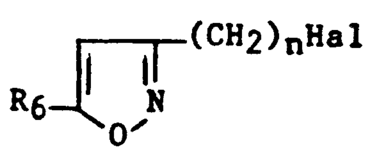

XI

where Hal is bromine or iodine. The compounds of Formula XI are in turn prepared by a reaction sequence involving conventional side-chain homologation reactions starting with a 5-$R_6$-isoxazole-3-carboxylic acid. This is illustrated by the procedures described hereafter in Example 7.

An alternative procedure for preparing a compound of Formula I where X is O comprises reacting a compound of Formula VII with an alkali metal derivative of a 3-R'-4-$R_4'$-5-methylisoxazole. Said alkali metal derivative is prepared in situ by treating the isoxazole with an organo-alkali-metal base under anhydrous conditions. A preferred organo-alkali-metal base is butyllithium or its complex with diisopropylamine.

The intermediates of Formula VII can be prepared by the reaction sequences set forth in the following flow sheet:

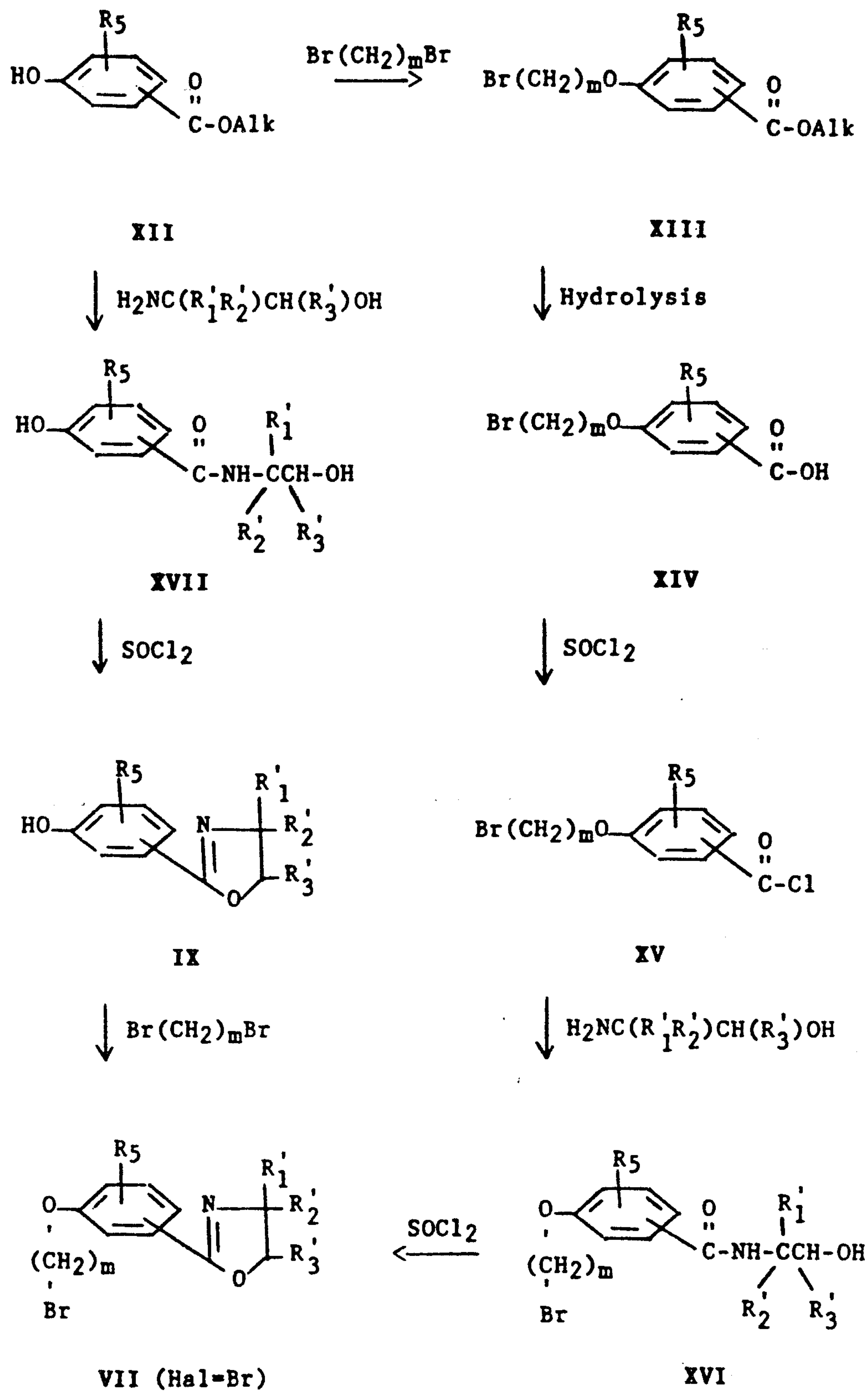


XII

XIII

XVII

XIV

IX

XV

VII (Hal=Br)

XVI

A hydroxybenzoate (XII, Alk = lower-alkyl) in the presence of a base reacts with an alkylene dibromide to form a bromoalkyl ether (XIII). The ester group is then hydrolyzed, preferably with a strong acid, to give the corresponding carboxylic acid (XIV). The latter is converted to its acid chloride (XV) which reacts with hydroxyethylamine or an alkylated or hydroxyalkylated derivative thereof to give an amide of the Formula XVI. The amide is then cyclized with thionyl chloride to give the desired intermediate of Formula VII (Hal = Br).

In an alternative approach, the ester XII is converted to the amide XVII and the latter cyclized to a phenolic dihydro-oxazole (IX). Etherification with an alkylene dibromide then gives VII (Hal = Br). The bromine atom can, if desired, be replaced by iodine by treating with a metallic iodide in an inert solvent.

A second alternative procedure for preparing a compound of Formula I where X is O comprises reacting a compound of Formula VIII with an alkali metal salt of a compound of Formula IX. The reaction takes place by heating the reactants in an inert solvent in the presence of an alkali metal base, e.g. potassium carbonate, at a temperature between about 50° and 150°C. The intermediate of Formula VIII is prepared by reacting an alkali metal derivative of a 3-R'-4-$R_4'$-5-methylisoxazole with an alkylene dihalide, Hal-$(CH_2)_m$-Hal, where m = n-1, in a reaction analogous to that where a compound of Formula VII is reacted with a 3-R'-4-$R_4'$-5-methylisoxazole.

The compounds of Formula I or II where one or more of R, $R_1$, $R_2$, $R_3$ and $R_4$ are lower-alkyl substituted by lower-alkanoyloxy, lower-alkoxy, chloro or N=Z are prepared from the corresponding compounds of Formula I or II where one or more of the recited R groups are hydroxyalkyl.

Esterification of a hydroxyalkyl compound by conventional procedures, as by reaction with a lower-alkanoic acid anhydride or halide gives the corresponding lower-alkanoyloxy derivative. The lower-alkanoyl groups preferably have from one to four carbon atoms.

Etherification of a hydroxyalkyl compound by conventional procedures, as by reaction with a lower-alkyl halide in the presence of a strong base, gives the corresponding lower-alkoxy derivative. The lower-alkoxy groups preferably have from one to four carbon atoms.

A hydroxyalkyl compound can be converted to a chloroalkyl compound by reaction with a reagent such as thionyl chloride or phosphorus trichloride, capable of replacing aliphatic hydroxy groups by chlorine.

The chloroalkyl compounds are in turn convertible to aminoalkyl compounds by reaction with ammonia or an amine, HN=Z. Compounds where HN=Z is lower-alkanoylamino are prepared by acylation of the compounds where HN=Z is $NH_2$ with a lower-alkanoyl halide or anhydride, lower-alkanoyl preferably having from 1 to 4 carbon atoms.

The compound of Formula I where R is $CH_3$, $R_1$, $R_2$, $R_3$, $R_4$ and $R_5$ are H, X is O, n is 7, and the dihydro-oxazole moiety is in the 4-position, was subjected to the fermentative enzymatic action of a variety of microorganisms. Two microorganisms, Aspergillus niger ($A_1$) and Trichothecium roseum ($T_1$) produced predominantly single oxidation products of the Formulas III (n = 7) and IV (n = 7), respectively, the structures being established by nuclear magnetic resonance data. It is contemplated that compounds of Formulas III and IV where n = 3-6 and 8-9 and/or $R_5$ is other than hydrogen can be obtained by similar microbiological oxidation of the respective compounds of Formula I.

The structures of the compounds of the invention were established by the modes of synthesis, by elementary analysis, and by infrared and nuclear magnetic resonance spectra.

The following examples will further illustrate the invention.

## Example 1

a) 4-(6-Bromohexyloxy)benzonitrile [X; Hal = Br, X = O, $R_5$ = H, m = 6, CN at 4-position].

A mixture of 23.8 g (0.2 mole) of 4-cyanophenol, 55.3 g (0.4 mole) of milled potassium carbonate, 97.6 g of 1,6-dibromohexane, 0.5 g of sodium iodide and 750 ml of acetone was stirred at reflux for two days. The solid was filtered off and the filtrate concentrated in vacuo. The residue was partitioned between water and methylene dichloride, and the organic phase was dried and concentrated. The residue was distilled to give 40 g of 4-(6-bromohexyloxy)benzonitrile, b.p. 150-160°C(0.05 mm).

b) 5-[7-(4-Cyanophenoxy)heptyl]-3-methylisoxazole [V; R' = $CH_3$, $R_4'$ and $R_5$ = H, X = O, n = 7, CN at 4-position].

To a suspension of 301 mg of lithium wire (1/4 inch portions) in 10 ml of tetrahydrofuran under nitrogen was added 6.72 ml of diisopropylamine and 3.44 ml of styrene while maintaining the temperature at 25°C. The mixture was stirred until all the lithium had dissolved (about four hours) and then cooled to -55°C. 3,5-Dimethylisoxazole (4.3 g) in 10 ml of tetrahydrofuran was then added dropwise and the mixture stirred for an hour at -55°C. 4-(6-Bromohexyloxy)benzonitrile (12 g) in 10 ml of tetrahydrofuran was then added dropwise over a period of one hour, and the mixture was allowed to

warm to room temperature and stirred for three days. The solvent was removed in vacuo, the residue treated with 5% ammonium chloride solution and extracted with ether. The ether extracts were dried and concentrated, and the residue subjected to high pressure liquid chromatography with ether-hexane (1:1) mixture to give 3.9 g of 5-[7-(4-cyanophenoxy)heptyl]-3-methylisoxazole, used directly in the next reaction.

c) 5-{7-[4-(4,5-Dihydro-2-oxazolyl)phenoxy]heptyl}-3-methylisoxazole [I; R = $CH_3$, $R_1$, $R_2$, $R_3$, $R_4$ and $R_5$ = H, X = O, n = 7, oxazole at 4-position].

A solution of 3.9 g of 5-[7-(4-cyanophenoxy)heptyl]-3-methylisoxazole in 10 ml of ethanol and 20 ml of ether was treated with gaseous hydrogen chloride at -70 °C until the solution was saturated. The solution was then allowed to warm to room temperature, allowed to stand for about 20 hours and the solvent removed in vacuo. The residue was crystallized from ethanol-ether to give 4.2 g of the corresponding ethyl imino-ester hydrochloride. The latter was mixed with 0.84 g of 2-hydroxyethylamine and heated at 120 °C for about three hours. The reaction mixture was cooled and crystallized from isopropyl acetate. A recrystallization from the same solid afforded 2.7 g of 5-{7-[4-(4,5-dihydro-2-oxazolyl)phenoxy]heptyl}-3-methylisoxazole, colorless solid, m.p. 89-90 °C.

A sample of the compound was treated with methanesulfonic acid in ethyl acetate solution to give the monomethanesulfonate salt, m.p. 134-135 °C.

Example 2

a) 4-(4-Bromobutyloxy)benzonitrile [X; Hal = Br, X = O, $R_5$ = H, m = 4, CN at 4-position] was prepared from 4-cyanophenol and 1,4-dibromobutane according to the procedure of Example 1, part (a), and was obtained in 52% yield; b.p. 155 °C(0.05 mm), m.p. 48-50 °C.

b) 5-[5-(4-Cyanophenoxy)pentyl]-3-methylisoxazole [V; R' = $CH_3$, $R_4'$ and $R_5$ = H, X = O, n = 5, CN at 4-position].

To a solution of 5.86 g of 3,5-dimethylisoxazole in 120 ml of dry tetrahydrofuran at -70 °C under nitrogen was added during 14 minutes 36 ml of n-butyllithium (1.7M in hexane). The mixture was stirred at -70 °C for 30 minutes, and then 15.2 g of 4-(4-bromobutyloxy)benzonitrile in 40 ml of tetrahydrofuran was added over a 15 minute period. The reaction mixture was stirred for 1.5 hours at -70 °C and then allowed to warm to room temperature and stirred for 2.5 hours longer. Evaporation of the solvent gave a residue which was treated with 300 ml of ethyl acetate, 150 ml of concentrated sodium chloride solution and 12 ml of hydrochloric acid. The material which was soluble in ethyl acetate was isolated, combined with material from another run of the same scale and subjected to high pressure liquid chromatography with ether-hexane (1:1) to give 20 g of 5-[5-(4-cyanophenoxy)pentyl]-3-methylisoxazole as a colorless solid, m.p. 57 °C.

c) 5-{5-[4-(4,5-Dihydro-2-oxazolyl)phenoxy]pentyl-3-methylisoxazole [I; R = $CH_3$, $R_1$, $R_2$, $R_3$, $R_4$ and $R_5$ = H, X = O, n = 5] was prepared by conversion of 5-[5-(4-cyanophenoxy)pentyl]-3-methylisoxazole to the corresponding ethyl imino-ester hydrochloride (m.p. 120-121 °C) and reaction of the latter with 2-hydroxyethylamine in accordance with the procedure of Example 1, part (c). The product was obtained in 68% yield as a colorless solid, m.p. 87-88 °C; monomethanesulfonate salt, m.p. 124-125 °C. The free base was obtained as a colorless solid, m.p. 95-96 °C.

Example 3

5-{7-[4-(4,5-Dihydro-4-methyl-2-oxazolyl)phenoxy]heptyl}-3-methylisoxazole [I; R and $R_1$ = $CH_3$, $R_2$, $R_3$, $R_4$ and $R_5$ = H, X = O, n = 7, oxazole at 4-position] was prepared from 5-[7-(4-cyanophenoxy)heptyl]-3-methylisoxazole following the procedure of Example 1, part (c) but substituting racemic 2-amino-1-propanol for the 2-hydroxyethylamine used therein. The product was obtained in about 65% yield as a colorless solid, m.p. 72 °C when recrystallized from isopropyl acetate.

Example 4

(—)-5-{7-[4-(4,5-Dihydro-4-methyl-2-oxazolyl)phenoxy]heptyl}-3-methylisoxazole [I; R and $R_1$ = $CH_3$, $R_2$, $R_3$, $R_4$ and $R_5$ = H, X = O, n = 7, oxazole at 4-position, levo-isomer] was prepared from 5-[7-(4-cyanophenoxy)heptyl]-3-methylisoxazole following the procedure of Example 1, part (c), but substituting methanol for ethanol in the imino-ester formation, and using L-2-amino-1-propanol in place of 2-hydroxyethylamine. Milder conditions (reflux in triethylamine) were used in the last step. The product was obtained in about 50% yield as a colorless solid, m.p. 71 °C, $[\alpha]_D^{25}$ (1% in ethanol) = -31.7 °.

Example 5

5-{7-[4-(4,5-Dihydro-4,4-dimethyl-2-oxazolyl)phenoxy]heptyl}-3-methylisoxazole [I; R, $R_1$ and $R_2$ = $CH_3$, $R_3$, $R_4$ and $R_5$ = H, X = O, n = 7, oxazole at 4-position] was prepared from 5-[7-(4-cyanophenoxy)heptyl]-3-methylisoxazole following the procedure of Example 1, part (c) but substituting 2-amino-2-methyl-1-propanol for the 2-hydroxyethylamine used therein. The product was obtained in about 65% yield as a colorless solid, m.p. 45-46°C when recrystallized from n-hexane.

It is further contemplated that by replacing the 2-hydroxyethylamine in Example 1, part (c) by a molar equivalent amount of tris(hydroxymethyl)aminomethane [tromethamine, $(HOCH_2)_3CNH_2$] there can be obtained 5-{7-[4-(4,5-dihydro-4,4-bishydroxymethyl-2-oxazolyl)phenoxy]heptyl}-3-methylisoxazole [I; R = $CH_3$, $R_1$ and $R_2$ = $CH_2OH$, $R_3$, $R_4$, and $R_5$ = H, X = O, n = 7].

Example 6

a) 4-Cyanophenylvinyl cyclopropyl ketone.

To a solution of 39.3 g of 4-cyanobenzaldehyde and 25.2 g of cyclopropyl methyl ketone in 60 ml of absolute ethanol was added 21 ml of 20% sodium hydroxide solution over a 25 minute period. The mixture was stirred for one hour at room temperature, cooled to 1°C and 40 ml of cold water added. The solid material was collected by filtration and triturated with 450 ml of methylene dichloride and 150 ml of water at room temperature. The aqueous phase was extracted with methylene dichloride and the combined organic layers dried and concentrated in vacuo. The residue was recrystallized from absolute ethanol to give 45.0 g of 4-cyanophenylvinyl cyclopropyl ketone, m.p. 104°C.

b) 4-Cyanophenylethyl cyclopropyl ketone.

A solution of 11.83 g of 4-cyanophenylvinyl cyclopropyl ketone in 200 ml of absolute ethanol containing 0.3 g of 10% palladium-on-carbon catalyst was hydrogenated at an initial pressure of 45 pounds per sq. in. for one hour. The catalyst was filtered off, and the product isolated from the filtrate and recrystallized from methanol to give 8.9 g of 4-cyanophenylethyl cyclopropyl ketone, m.p. 76°C.

c) 4-Cyanophenylethyl cyclopropyl carbinol.

To a solution of 30.9 g of 4-cyanophenylethyl cyclopropyl ketone in 90 ml of absolute ethanol was added 1.48 g of sodium borohydride, and the mixture was stirred at room temperature for three hours. The product isolated from the reaction still contained unreacted starting material, so the material was redissolved in 90 ml of ethanol and treated with 0.7 g additional sodium borohydride for three hours. The product obtained by evaporation of the solvent, trituration of the residue with methylene dichloride and water, and isolation of the product from the organic phase, gave 31.0 g of 4-cyanophenylethyl cyclopropyl carbinol as an oil which crystallized to a colorless solid, m.p. 70-71°C.

d) 4-(6-Bromohex-3-enyl)benzonitrile.

To a solution of 9.8 g of 4-cyanophenylethyl cyclopropyl carbinol in 140 ml of ether was added 4.24 g of lithium bromide and 3 ml of 2,4,6-collidine. The mixture was cooled to -60°C and 9.8 g of phosphorus tribromide was added over a five minute period. The reaction mixture was allowed to warm to 0°C, kept at that temperature for two hours and then allowed to warm to 18°C. Collidine (18 ml) was added, and after 15 minutes of stirring, the mixture was poured into 200 ml of water and 100 ml of ether. The ether extracts were washed with dilute aqueous sulfuric acid and water, dried over anhydrous magnesium sulfate and concentrated to a volume of 150 ml. Zinc bromide (11.6 g) was then added with cooling, and the mixture was stirred at room temperature for 29 hours. The ether solution was washed with water, dried and concentrated to give 12.5 g of 4-(6-bromohex-3-enyl)benzonitrile as a yellow oil.

e) 4-(6-Bromohexyl)benzonitrile.

A solution of 10.5 g of 4-(6-bromohex-3-enyl)benzonitrile in 200 ml of absolute ethanol was hydrogenated in the presence of 0.25 g of platinum oxide catalyst. Isolation of the product afforded 10.4 g of 4-(6-bromohexyl)benzonitrile as a yellow oil which was distilled at 168-170°C(0.01 mm) to produce the compound as a colorless oil which solidified upon cooling.

f) 5-[7-(4-Cyanophenyl)heptyl]-3-methylisoxazole [V; R = $CH_3$, $R_5$ = H, X = single bond, n = 7, CN at 4-position] was prepared from 4-(6-bromohexyl)benzonitrile and the lithium derivative of 3,5-dimethylisoxazole according to the procedure of Example 2(b). The crude product was chromatographed on magnesium silicate (Florisil) using the solvent series hexane:ether:methanol for elution. Ether—hexane 30:70 and 40:60 brought out the desired 5[7-(4-cyanohexyl)heptyl]-3-methylisoxazole, obtained as a colorless solid, m.p. 61°C, when recrystallized from ether.

g) 5-{7-[4-(4,5-Dihydro-2-oxazolyl)phenyl]heptyl}-3-methylisoxazole [I; R = $CH_3$, $R_1$, $R_2$, $R_3$, $R_4$ and $R_5$ = H, X = single bond, n = 7, CN at 4-position].

A suspension of 5.08 g of 5-[7-(4-cyanophenyl)heptyl]-3-methylisoxazole in 35 ml of dry methanol at -5—0 ° C was saturated with hydrogen chloride gas (55 minutes). The mixture was kept cold for two days, then concentrated in vacuo at 25-30 ° C and the residue stirred with 70 ml ether and cooled. The product was collected and dried to give 6.1 g of the methyl imino-ester hydrochloride, m.p. 116 ° C(decompn.).

A mixture of 3.5 g of the imino-ester hydrochloride, 1 ml of triethylamine, 0.67 g of 2-aminoethanol and 15 ml of ethylene dichloride was stirred at room temperature for two hours. Additional triethylamine (1 ml) was then added and the mixture heated at reflux for one hour. The reaction mixture was cooled, filtered, diluted with 50 ml of methylene dichloride and washed with water. The water layer was backwashed with methylene chloride, and the combined organic layers were dried over anhydrous magnesium sulfate and concentrated to dryness. There was thus obtained 2.8 g of 5-{7-[4-(4,5-dihydro-2-oxazolyl)phenyl]heptyl}-3-methylisoxazole, colorless solid, m.p. 66-67 ° C. Recrystallization from hexane caused no change in melting point.

Example 7

a) 3-Hydroxymethyl-5-methylisoxazole was prepared from 93.4 g of methyl 5-methylisoxazole-3-carboxylate and 62.5 g of sodium borohydride in 1200 ml of t-butyl alcohol and 600 ml of methanol. Isolation of the product and distillation gave 59.2 g of 3-hydroxymethyl-5-methylisoxazole, b.p. 68-70 ° C(0.05 mm).

b) 3-Chloromethyl-5-methylisoxazole.

To a solution of 119.6 g of 3-hydroxymethyl-5-methylisoxazole in 600 ml of ether was slowly added 155 ml of thionyl chloride in 200 ml of ether over a five hour period. The solution was concentrated to an oily residue which was distilled to give 121.2 g of 3-chloromethyl-5-methylisoxazole, b.p. 70-71 ° C(11 mm).

c) 5-Methyl-3-isoxazolepropanoic acid.

To a stirred suspension of 78.5 g of sodium hydride in 1 liter of tetrahydrofuran under nitrogen was added in portions 261 g of diethyl malonate. When evolution of hydrogen had ceased, 108 g of 3-chloromethyl-5-methylisoxazole was added and the reaction mixture was heated at reflux for four hours. A portion of the tetrahydrofuran (800 ml) was distilled off and 1 liter of 5% sodium hydroxide solution was added to the remaining mixture which was then heated at reflux for three hours and allowed to stand at room temperature for three days. The reaction mixture was filtered and the filtrate extracted with hexane. The aqueous layer was acidified with concentrated hydrochloric acid and extracted repeatedly with ethyl acetate. The ethyl acetate was removed in vacuo, 100 ml of pyridine added to the residue, and the mixture heated at reflux for three hours until evolution of carbon dioxide ceased. The mixture was concentrated in vacuo and the residue acidified with 6N hydrochloric acid and cooled. The solid which separated was collected and dissolved in methylene dichloride. The layers were separated and the methylene dichloride layer concentrated in vacuo. The residual solid was slurried with isopropyl acetate—hexane to give 75.4 g of 5-methyl-3-isoxazolepropanoic acid, m.p. 82-84 ° C.

d) Methyl 5-methyl-3-isoxazolepropanoate.

A mixture of 75.4 g of 5-methyl-3-isoxazolepropanoic acid, 150 ml of boron trifluoride etherate and 400 ml of methanol was heated at reflux for eight hours. The reaction mixture was concentrated in vacuo, made basic with sodium bicarbonate solution and extracted with methylene dichloride. The extracts were concentrated in vacuo and the residue distilled at 90-100 ° C (0.05 mm) to give 73 g of methyl 5-methyl-3-isoxazolepropanoate which crystallized to a solid, m.p. 54-55 ° C.

e) 5-Methyl-3-(3-hydroxypropyl)isoxazole.

To a suspension of 7.6 g of lithium aluminum hydride in 250 ml of tetrahydrofuran was added a solution of 64.9 g of methyl 5-methyl-3-isoxazolepropanoate in 100 ml of tetrahydrofuran. The reaction mixture was stirred at reflux for three hours, then cooled and 15.2 ml of water in 30 ml of tetrahydrofuran added. The mixture was filtered and the filtrate concentrated in vacuo. The residue was distilled to give 44.1 g of 5-methyl-3-(3-hydroxypropyl)isoxazole, b.p. 84-85 ° C(0.1 mm).

f) 5-Methyl-3-(3-bromopropyl)isoxazole.

Bromine (33.8 g) was added to a suspension of 55.5 g of triphenylphosphine in 400 ml of acetonitrile. The mixture was stirred for 30 minutes and concentrated in vacuo to remove the solvent. To the residue was added 200 ml of dimethylformamide, and with stirring 29.8 g of 5-methyl-3-(3-hydroxypropyl)isoxazole was added. An exothermic reaction ensued and the solid materials dissolved to form an orange solution which was poured into water and extracted with methylene dichloride. The methylene dichloride extracts were concentrated and the residue distilled to give 34.1 g of 5-methyl-3-(3-bromopropyl)isoxazole, b.p. 115-125 ° C(0.05 mm).

g) 3-(4-Carboxybutyl)-5-methylisoxazole was prepared from 5-methyl-3-(3-bromopropyl)isoxazole and diethyl malonate according to the procedure of part (c) above, and was obtained in 56% yield as a colorless solid, m.p. 58-60 ° C when recrystallized from carbon tetrachloride.

h) 3-(5-Hydroxypentyl)-5-methylisoxazole was prepared by reduction of 3-(4-carboxybutyl)-3-methylisoxazole with lithium aluminum hydride according to the procedure of part (e) above, and was obtained in 84% yield as an oil, b.p. 115-125 ° C(0.1 mm).

i) 3-(5-Bromopentyl)-5-methylisoxazole was prepared by reacting 3-(5-hydroxypentyl)-5-methylisoxazole with bromine and triphenylphosphine according to the procedure of part (f) above, and was obtained in 77% yield as an oil, b.p. 140-150 ° C (0.05 mm).

j) 3-[5-(4-Cyanophenoxy)pentyl]-5-methylisoxazole [VI; $R_5$ = H, $R_6$ = $CH_3$, n = 5, CN at 4-position].

A mixture of 5.1 g of 4-cyanophenol, 10 g of 3-(5-bromopentyl)-5-methylisoxazole, 8 g of potassium carbonate, 1 g of potassium iodide and 75 ml of acetonitrile was heated at reflux for 24 hours. The product was isolated and distilled, first at 115-200 ° C(0.1 mm) and then at 160-190 ° C(0.05 mm) to yield a yellow oil which crystallized upon cooling. Recrystallization from hexane—ether afforded 6.2 g of 3-[5-(4-cyanophenoxy)pentyl]-5-methylisoxazole, m.p. 61-62 ° C.

k) 3-{5-[4-(4,5-Dihydro-2-oxazolyl)phenoxy]pentyl}-5-methylisoxazole [II; $R_1$, $R_2$, $R_3$ and $R_5$ = H, $R_6$ = $CH_3$, n = 5, oxazole at 4-position].

A solution of 6.2 g of 3-[5-(4-cyanophenoxy)pentyl]-5-methylisoxazole in 15 ml of absolute ethanol and 30 ml of ether was cooled to -60 ° C and saturated with hydrogen chloride gas over a 45 minute period. The reaction mixture was warmed to room temperature, allowed to stand for three days and then concentrated in vacuo to a solid residue. The latter was recrystallized from ethanol by addition of ether to give 9.6 g of the ethyl imino-ester hydrochloride. The latter was dissolved in 25 ml of chloroform, 3.5 ml of triethylamine was added, and the mixture stirred for one hour. The solution was washed with water, dried over magnesium sulfate and concentrated in vacuo. To the residual oil was added 1.4 g of 2-hydroxyethylamine and the mixture was heated at 115-120 ° C for 1.5 hours. The reaction mixture was cooled and the solid product recrystallized three times from isopropyl acetate to give 4.4 g of 3-{5-[4-(4,5-dihydro-2-oxazolyl)phenoxy]pentyl}-5-methylisoxazole, m.p. 91-92 ° C.

Example 8

a) Ethyl 4-(6-bromohexyloxy)benzoate [XIII; Alk = $C_2H_5$, $R_5$ = H, m = 6].

To a solution of 232 g of ethyl 4-hydroxybenzoate [XII; Alk = $C_2H_5$] in 1.2 liters of dimethylsulfoxide was added 100 g of potassium hydroxide. The mixture was stirred for five minutes, 678 g of 1,6-dibromohexane was then added, and the mixture stirred for four hours during which an exothermic reaction occurred (max. temp. 46 ° C). The reaction mixture was added to 1500 ml of water and extracted three times with 800 ml of cyclohexane. The cyclohexane layer was washed with 800 ml of water, 200 ml 2N potassium hydroxide and 800 ml concentrated sodium chloride solution, then filtered and concentrated in vacuo. The residue was triturated with ether to give 328.1 g of ethyl 4-(6-bromohexyloxy)-benzoate.

b) 4-(6-Bromohexyloxy)benzoic acid [XIV; $R_5$ = H, m = 6].

Concentrated sulfuric acid (1036 ml) was added gradually to 338 ml of water, followed by 622 g of ethyl 4-(6-bromohexyloxy)benzoate. The mixture was heated at 100-110 ° C for 35 minutes and then poured onto 2.5 kg ice with stirring. The mixture was treated with 2520 ml of ammonium hydroxide to bring the pH to 6.0. The product was collected by filtration, washed with water and hexane, and dried in an oven to give 452 g of 4-(6-bromohexyloxy)benzoic acid.

c) 4-(6-Bromohexyloxy)benzoyl chloride [XV; $R_5$ = H, m = 6].

A mixture of 452 g of 4-(6-bromohexyloxy)benzoic acid and 1000 g of thionyl chloride was stirred at room temperature for about 20 hours. The reaction mixture was concentrated in vacuo and residual thionyl chloride removed by repeated addition of toluene and concentration in vacuo. The oily product comprising 4-(6-bromohexyloxy)benzoyl chloride (450 ml) was used directly in the next reaction.

d) 4-(6-Bromohexyloxy)-N-(2-hydroxyethyl)benzamide [XVI; $R_1'$, $R_2'$, $R_3'$, and $R_5$ = H, m = 6].

To a stirred solution of 300 ml of 2-hydroxyethylamine in 1.2 liter of dimethylformamide at 0 ° C was added dropwise 450 ml of 4-(6-bromohexyloxy)benzoyl chloride in 50 ml of toluene over a 30 minute period. After an additional 30 minutes of stirring, 48% hydrobromic acid (about 200 ml) was added until the pH reached 5-5.5, and the mixture was diluted with 1200 ml of water and extracted with isopropyl acetate (total of 2500 ml). The extracts were washed with water and saturated sodium chloride solution,

filtered, and concentrated to a volume of about 1 liter. The mixture was cooled and the solid which separated was collected by filtration and dried at 40°C to give 332.7 g of 4-(6-bromohexyloxy)-N-(2-hydroxyethyl)benzamide.

e) 2-[4-(6-Bromohexyloxy)phenyl]-4,5-dihydro-oxazole [VII; Hal = Br, $R_1'$, $R_2'$, $R_3'$ and $R_5$ = H, m = 6, oxazole at 4-position].

To 100 g of 4-(6-bromohexyloxy)-N-(2-hydroxyethyl)benzamide was added dropwise 112 g (75 ml) of thionyl chloride over a 20 minute period. The mixture was stirred for 45 minutes and then diluted with several volumes of ether. A solid product separated which was collected and rinsed with ether to give 90 g of 2-[4-(6-bromohexyloxy)phenyl]-4,5-dihydro-oxazole.

f) 5-{7-[4-(4,5-Dihydrdo-2-oxazolyl)phenoxy]hepyl}-3-methylisoxazole [I; R = $CH_3$, $R_1$, $R_2$, $R_3$, $R_4$ and $R_5$ = H, X = 0, n = 7, oxazole at 4-position].

To a stirred solution of 6.0 ml of diisopropylamine in 50 ml of tetrahydrofuran at 0°C was added 22 ml of 2.1M n-butyllithium in hexane over a period of 20 minutes. The mixture was stirred 30 minutes at 5°C, then cooled to -55°C, and 4.5 g of 3,5-dimethylisoxazole was added dropwise during 15 minutes. The resulting slurry was stirred for 30 minutes at -60°C and 12.4 g of 2-[4-(6-bromohexyloxy)phenyl]-4,5-dihydro-oxazole in 35 ml of tetrahydrofuran was then added dropwise. After the addition was complete, the mixture was stirred for one hour during which the temperature rose to -40°C. The reaction mixture was allowed to warm to room temperature, 75 ml of water was then added dropwise, end the mixture was extracted with ethyl acetate. The ethyl acetate extracts were dried and concentrated in vacuo, and the residue triturated with ether. The ether insoluble fraction (10.5) consisted essentially of 5-{7-[4-(4,5-dihydro-2-oxazolyl)phenoxy]heptyl}-3-methylisoxazole, identical with the product of Example 1, part (c).

Example 9

a) N-(2-Hydroxyethyl)-4-hydroxybenzamide [XVII; $R_1'$, $R_2'$, $R_3'$ and $R_5$ = H, OH at 4-position].

A mixture of 80 g of methyl 4-hydroxybenzoate and 120 ml of ethanolamine was heated at 150°C for five hours during which time 14.2 ml of methanol was distilled off. The excess ethanolamine was removed in vacuo, and the residue was treated with two 150 ml portions of chloroform. The chloroform was removed in vacuo and the residual oil dissolved in acetone from which the product crystallized to give 45.3 g of N-(2-hydroxyethyl)-4-hydroxybenzamide.

b) 4,5-Dihydro-2-(4-hydroxyphenyl)oxazole [IX; $R_1'$, $R_2'$, $R_3'$ and $R_5$ = H, OH at 4-position].

Thionyl chloride (160 ml) was added to 40 g of N-(2-hydroxyethyl)-4-hydroxybenzamide with evolution of gas. The reaction mixture was ultrasonicated for 1.75 hours, then cooled and diluted with ether. The resulting solid product was collected by filtration, washed with ether and dried overnight in a vacuum oven at 40°C to give 42.5 g of 4,5-dihydro-2-(4-hydroxyphenyl)oxazole in the form of its hydrochloride salt.

c) 5-(7-Bromoheptyl)-3-methylisoxazole [VIII; R = $CH_3$, n = 7, Hal = Br].

To a stirred solution of 46.1 ml of diisopropylamine in 100 ml of tetrahydrofuran at 0-5°C was added 126 ml of 2.6M n-butyllithium in hexane over a period of about 20 minutes. The mixture was cooled to -50°C, 31.95 g of 3,5-dimethylisoxazole was added and the mixture stirred for 30 minutes. The latter mixture was cooled to -78°C and 101.6 ml of 1,6-dibromohexane was added dropwise. After the addition was complete, the temperature of the reaction mixture was allowed to rise to room temperature and kept there for about 40 hours. Saturated aqueous ammonium chloride solution was then added and the mixture extracted with ethyl acetate. The ethyl acetate extracts were dried over anhydrous magnesium sulfate and concentrated in vacuo. The residue was distilled to remove excess dibromide (b.p. 50°C, 0.5 mm) and suspended in hexane. The suspension was decolorized with charcoal, filtered and cooled in a refrigerator overnight. The mixture was filtered and the filtrate concentrated in vacuo to give 57 g of 5-(7-bromoheptyl)-3-methylisoxazole as a colorless oil.

d) 5-{7-[4-(4,5-Dihydro-2-oxazolyl)phenoxy]heptyl}-3-methylisoxazole [I; R = $CH_3$, $R_1$, $R_2$, $R_3$, $R_4$ and $R_5$ = H, X = O, n = 7, oxazole at 4-position].

Sodium iodide (6.43 g, 0.043 m) was added to a solution of 9.36 g (0.036 m) of 5-(7-bromoheptyl)-3-methylisoxazole in 100 ml of acetonitrile, and the mixture was stirred at reflux for two hours and then cooled to room temperature. Potassium carbonate (11.8 g, 0.086 m) and 10.12 g (0.043 m) of 4,5-dihydro-2-(4-hydroxyphenyl)oxazole hydrochloride were then added, and the reaction mixture was heated at refux for 24 hours. The reaction mixture was cooled, poured into water and extracted with three 75 ml portions of ethyl acetate. The extracts were dried over anhydrous magnesium sulfate and concentrated in vacuo. The solid residue was recrystallized from acetonitrile to give a first crop consisting of recovered

4,5-dihydro-2-(4-hydroxyphenyl)oxazole (1.0 g) and a second crop consisting of the desired product, 5-{7-[4-(4,5-dihydro-2-oxazolyl)phenoxy]heptyl}-3-methylisoxazole, m.p. 85-86°C, identical with the compound obtained in Example 1, part (c) and Example 8, part (f), as determined by thin layer chromatographic analysis.

Example 10

a) N-(2-Hydroxyethyl)-4-hydroxybenzamide [XVII; $R_1'$, $R_2'$, $R_3'$ and $R_5$ = H, OH at 4-position].

A 5 liter, three-necked flask, equipped with a Dean-Stark trap, mechanical stirrer and thermometer was charged with 608 g (4.0 moles) of methyl p-hydroxybenzoate and 488 g (8.0 moles) of 2-aminoethanol. The mixture, when stirred and heated to 135°C, gave a clear solution. After heating the mixture for 2.5 hrs at 135-140°C, 114 ml of methanol was collected in the Dean-Stark trap. The solution was cooled to 70°C at which time some thickening occurred. The solution was treated with 2 liters of 2N hydrochloric acid and allowed to cool. A white crystalline precipitate formed and the mixture was stirred and cooled to 0°C to complete crystallization. The solid was filtered and dried in vacuo at 65°C overnight. Wt. = 634.6 g (87.6%), m.p. 156-157°C.

b) 4,5-Dihydro-2-(4-hydroxyphenyl)oxazole [IX; $R_1'$, $R_2'$, $R_3'$ and $R_5$ = H, OH at 4-position].

A 22 liter, three-necked flask was charged with 634 g (3.5 moles) of the β-hydroxyamide of part (a) and 5.2 liters of isopropyl acetate. The flask was cooled externally in a bath with tap water (10-15°C) and 390 ml (5.25 moles) of thionyl chloride was added to the stirred suspension over 45 minutes. A mild exotherm was apparent, but the temperature was maintained between 25 and 30°C. After stirring 2 hr at ambient temperature, the suspension was filtered and the cake washed with isopropyl acetate. After air drying for two hours, the solid was transferred to a 22 liter flask and dissolved in 1.4 liters of water. The solution was treated with saturated sodium bicarbonate solution until slightly basic. A heavy white precipitate formed during the addition. The suspension was filtered and the resulting white solid was washed with cold water and dried overnight in vacuo at 65°C. A total of 522 g (91.5%) of the 4,5-dihydro-2-(4-hydroxyphenyl)oxazole, m.p. 209-211°C, was obtained.

c) 2-[4-(6-Bromohexyloxy)phenyl]-4,5-dihydro-oxazole [VII;$R_1'$, $R_2'$, $R_3'$ and $R_5$ = H, Hal = Br, m = 6, oxazole at 4-position].

A stirring suspension of 163 g (1.0 mole) of 4,5-dihydro-(4-hydroxyphenyl)oxazole and 276 g (2.0 mole) milled potassium carbonate in 750 ml acetonitrile was heated to reflux in a 5 liter round-bottomed flask. A total of 457.5 ml (3.0 moles) of 1,6-dibromohexane was added all at once and reflux continued for 1 hour. The reaction mixture was filtered and the cake of organic salts washed with 100 ml of acetonitrile. The filtrate was evaporated on a rotary evaporator (bath temperature <40°C) and the resulting residue slurried in 1.6 liters of hexane and chilled to 0°C. The resulting white solid was filtered and washed with 500 ml cold hexane. The recovery was 263 g (80%) of white solid. A total of 535 g of crude product was slurried in 1.1 liter of refluxing tert-butyl methyl ether. The mixture was allowed to cool to 30°C and filtered with suction. The filtrate was cooled to 0°C and the product crystallized. The solid was filtered off and dried to yield 372 g (70% recovery) of 2-[4-(6-bromohexyloxy)phenyl]-4,5-dihydro-oxazole, m.p. 79-81°C. The crude product can also be recrystallized from acetonitrile. This removes excess dibromohexane and most of an undesired byproduct formed by reaction of 1 mole of dibromohexane with 2 moles of 4,5-dihydro-2-(4-hydroxyphenyl)oxazole, namely 1,6-bis-[4-(4,5-dihydro-2-oxazolyl)phenoxy]hexanet, m.p. 174-175°C when isolated and purified.

d) 5-{7-[4-(4,5-Dihydro-2-oxazolyl)phenoxy]heptyl}-3-methylisoxazole [I; R = $CH_3$, $R_1$, $R_2$, $R_3$, $R_4$ and $R_5$ = H, X = O, n = 7, oxazole at 4-position].

To a stirring solution of 48.6 g (0.48 moles) diisopropylamine in 520 ml tetrahydrofuran (THF) at -5°C (ice/acetone bath) was added 185 ml of 2.6M n-butyllithium (0.48 moles) under nitrogen. The addition was complete after 30 minutes and the pale yellow solution was maintained at -5° to +5°C for an additional 30 minutes. The ice/acetone bath was replaced with Dry Ice/acetone and when the internal temperature reached -55°C, 46.6 g (0.48 moles) 3,5-dimethylisoxazole was added dropwise over 20 minutes. This solution was allowed to stir an additional 30-40 minutes at -55°C or lower. A solution containing 135 g (0.41 moles) 2-[4-(6-bromohexyloxy)phenyl]-4,5-dihydro-oxazole dissolved in 400 ml THF was added dropwise (via nitrogen pump) over 35 minutes. The temperature during the addition was kept below -50°C by carefully controlling the rate of the addition. The heavy suspension was stirred for an additional hour. The Dry Ice bath was removed and the reaction was quenched by dropwise addition of 10 ml water. The reaction mixture was allowed to warm to about 5-10°C and then poured into 1 liter water and 1 liter ethyl acetate. The aqueous layer was set aside and the organic layer was washed once with water and brine. The extract was dried over magnesium sulfate and evaporated to near dryness

under water vacuum. The crude crystalline product was dissolved in 600 ml warm acetonitrile, filtered thru a pad of solka floc and allowed to crystallize at room temperature. After two hours, the heavy crystalline precipitate was cooled to 10°C and filtered to give 108 g (76%) 5-{7-[4-(4,5-dihydro-2-oxazolyl)phenoxy]heptyl}-3-methylisoxazole, m.p. 97-98°C, after drying 24 hrs at 60°C.

An effective way of removing traces of the byproduct produced in part (c) carried through to the final product is by recrystallization from ethanol (solution formation at 50°C, crystallization at 40°C and collection at 35°C).

Example 11

5-{7-[4-(4,5-Dihydro-2-oxazolyl)phenoxy]heptyl}-3-isoxazolemethanol [III; $R_5$ = H, n = 7].

5-{7-[4-(4,5-Dihydro-2-oxazolyl)phenoxy]heptyl}-3-methylisoxazole (Example 1c) (1 g) was added to each of three 10-liter fermentation tanks containing a culture of *Aspergillus niger* ($A_1$) in soy-dextrose medium. After 24 hours, thin layer chromatography showed essentially complete conversion to a more polar product. The total fermentation brews were extracted with two volumes each of dichloromethane. These were combined and concentrated *in vacuo*. The concentrate was washed with 0.05N sodium hydroxide and with water, leaving an oily material upon removal of remaining solvent. Several crystallizations from ethyl acetate and from acetone yielded 330 mg of 5-{7-[4-(4,5-dihydro-2-oxyazolyl]phenoxy]heptyl}-3-isoxazolemethanol, colorless needles, m.p. 122-124°C. The structure was established by nmr, ir and uv spectra.

Example 12

The procedure of Example 11 was repeated but using *Trichothecium roseum* ($T_1$) as the microorganism. From 1.5 g of starting material there was obtained, using preparative silica gel thin layer chromatography for purification, 200 mg of α-{6-[4-(4,5-dihydro-2-oxazolyl)phenoxy]hexyl}-3-methyl-5-isoxazolemethanol (IV; $R_5$ = H, n = 7), colorless crystals, m.p. 132-133°C; structure established by mass spectrum and nmr spectrum.

Example 13

a) N-(2-Hydroxyethyl)-2-chloro-4-hydroxybenzamide [XVII; $R_1'$, $R_2'$, $R_3'$, = H, $R_5$ = 2-Cl, OH at 4-position] was prepared from methyl 3-chloro-4-hydroxybenzoate and ethanolamine according to the procedure of Example 9, part (a). The product thus obtained had the m.p. 148-150°C.

b) 4,5-Dihydro-2-(3-chloro-4-hydroxyphenyl)oxazole [IX; $R_1'$, $R_2'$, $R_3'$, = H, $R_5$ = 3-Cl, oxazole at 4-position] was prepared by reacting N-(2-hydroxyethyl)-2-chloro-4-hydroxybenzamide with thionyl chloride according to the procedure of Example 9, part (a). The product was obtained in the form of its hydrochloride salt, m.p. 153-155°C.

c) 5-{7-[2-Chloro-4-(4,5-dihydro-2-oxazolyl)phenoxy]heptyl}-3-methylisoxazole [I; R = $CH_3$, $R_1$, $R_2$, $R_3$ and $R_4$ = H, $R_5$ = 2-Cl, X = O, n = 7, oxazole at 4-position] was prepared from 5-(7-bromoheptyl)-3-methylisoxazole (Example 9, part c) and 4,5-dihydro-2-(3-chloro-4-hydroxyphenyl)oxazoleaccording to the procedure of Example 9, part (d), and was obtained in the form of a colorless solid, m.p. 120-120.5°C when recrystallized from methanol and further purified by chromatography.

Example 14

a) 5-(6-Bromohexyl)-3-methylisoxazole [VIII; R = $CH_3$, n = 6, Hal = Br] was prepared from 1,5-dibromopentane and 3,5-dimethylisoxazole according to the procedure of Example 9, part (c), and was obtained as a yellow oil in 46% yield.

b) 5-{6-[4-(4,5-Dihydro-2-oxazolyl)phenoxy]hexyl}3-methylisoxazole [I; R = $CH_3$, $R_1$, $R_2$, $R_3$, $R_4$ and $R_5$ = H, X = O, n = 6, oxazole at 4-position] was prepared by reacting 5-(6-bromohexyl)-3-methylisoxazole and 4,5-dihydro-2-(4-hydroxyphenyl)oxazole (free base, m.p. 200-202°C) according to the procedure of Example 9, part (d), and was obtained as a colorless solid, m.p. 88°C when recrystallized from hexane.

Example 15

a) 5-(5-Bromopentyl)-3-methylisoxazole [VIII; R = $CH_3$, n = 5, Hal = Br] was prepared from 1,4-dibromobutane and 3,5-dimethylisoxazole according to the procedure of Example 9, part (c), and was obtained as an oil in 52% yield.

b) 5-{5-[2-Chloro-4-(4,5-dihydro-2-oxazolyl)phenoxy]pentyl}-3-methylisoxazole [I; R = $CH_3$, $R_1$, $R_2$, $R_3$ and $R_4$ = H, $R_5$ = 2-Cl, X = O, n = 5, oxazole at 4-position] was prepared from 5-(5-bromopentyl)-3-methylisoxazole and 4,5-dihydro-2-(3-chloro-4-hydroxyphenyl)oxazole (Example 13b) according to the procedure of Example 9, part (d), and was obtained in the form of a colorless solid, m.p. 102-104 °C when recrystallized from isopropyl alcohol.

Example 16

5-{6-[2-chloro-4-(4,5-dihydro-2-oxazolyl)phenoxy]hexyl}-3-methylisoxazole [I; R = $CH_3$, $R_1$, $R_2$, $R_3$ and $R_4$ = H, $R_5$ = 2-Cl, X = O, n = 6, oxazole at 4-position] was prepared from 5-(6-bromohexyl)-3-methylisoxazole (Example 14a) and 4,5-dihydro-2-(3-chloro-4-hydroxyphenyl)oxazole (Example 13b) according to the procedure of Example 9, part (b), and was obtained in the form of a colorless solid, m.p. 64.5-65.5 °C when recrystallized from ether— pentane.

Example 17

a) 3,5-Dihydro-2-(3-chloro-4-hydroxyphenyl)-4-methyloxazole [IX; $R_1'$ = $CH_3$, $R_2'$ and $R_3'$ = H, $R_5$ = 3-Cl, OH at 4-position] was prepared from methyl 3-chloro-4-hydroxybenzoate and 2-aminopropanol, and cyclization of the resulting N-(2-hydroxypropyl)-4-hydroxybenzamide with thionyl chloride according to the procedures of Example 9, parts (a) and (b), and was obtained in 60% yield as a colorless solid, m.p. 176-178 °C when recrystallized from acetone.

b) 5-{7-[2-Chloro-4-(4,5-dihydro-4-methyl-2-oxazolyl)phenoxy]heptyl}-3-methylisoxazole [I; R and $R_1$ = $CH_3$, $R_2$, $R_3$ and $R_4$ = H, $R_5$ = 2-Cl, X = O, n = 7, oxazole at 4-position] was prepared from 3,5-dihydro-2-(3-chloro-4-hydroxyphenyl)-4-methyloxazole and 5-(7-bromoheptyl)-3-methylisoxazole according to the procedure of Example 9, part (d), and was obtained in 69% yield as a colorless solid, m.p. 80-82 °C when recrystallized first from cyclohexane and then from tertiary-butyl methyl ether.

Example 18

a) 5-(8-Bromooctyl)-3-methylisoxazole [VIII; R = $CH_3$, n = 8, Hal = Br] was prepared from 1,7-dibromoheptane and 3,5-dimethylisoxazole according to the procedure of Example 9, part (c) and was obtained in 52% yield as a yellow oil.

b) 5-{8-[4-(4,5-Dihydro-2-oxazolyl)phenoxy]octyl}-3-methylisoxazole [I; R = $CH_3$, $R_1$, $R_2$, $R_3$, $R_4$ and $R_5$ = H, X = O, n = 8, oxazole at 4-position] was prepared from 5-(8-bromooctyl)-3-methylisoxazole and 4,5-dihydro-2-(4-hydroxyphenyl)oxazole according to the procedure of Example 9, part (d), and was obtained as a colorless solid, m.p. 73.5-74.5 °C when recrystallized from pentane.

Example 19

a) 4,5-Dihydro-2-(3-fluoro-4-hydroxyphenyl)oxazole [IX; $R_1'$ , $R_2'$ and $R_3'$ = H, $R_5$ = 3-F, oxazole at 4-position] was prepared by reacting methyl 3-fluoro-4-hydroxybenzoate with ethanolamine, and treating the resulting N-(2-hydroxyethyl)-3-fluoro-4-hydroxybenzamide with thionyl chloride, according to the procedures of Example 9, parts (a) and (b), and was obtained in 41% yield as a colorless solid, m.p. 201-203 °C, when recrystallized from tetrahydrofuran.

b) 5-{7-[4-(4,5-Dihydro-2-oxazolyl)-2-fluorophenoxy]heptyl}-3-methylisoxazole [I; R = $CH_3$, $R_1$, $R_2$, $R_3$ and $R_4$ = H, $R_5$ = 2-F, X = O, n = 7, oxazole at 4-position] was prepared from 4,5-dihydro-2-(3-fluoro-4-hydroxyphenyl)oxazole and 5-(7-bromoheptyl)-3-methylisoxazole according to the procedure of Example 9, part (d), and was obtained in 55% yield as a colorless solid, m.p. 83-84 °C, when recrystallized first from methanol and then from tertiarybutyl methyl ether.

Example 20

5-{5-[4-(4,5-Dihydro-2-oxazolyl)-2-fluorophenoxy]pentyl}-3-methylisoxazole [I; R = $CH_3$, $R_1$, $R_2$, $R_3$ and $R_4$ = H, $R_5$ = 2-F, X = O, n = 5, oxazole at 4-position] was prepared from 4,5-dihydro-2-(3-fluoro-4-hydroxyphenyl)oxazole (Example 19a) and 5-(5-bromopentyl)-3-methylisoxazole (Example 15a), according to the procedure of Example 9, part (d), and was obtained in 51% yield as a colorless solid, m.p. 95-96°C, when recrystallized from tertiarybutyl methyl ether.

Example 21

a) 5-(4-Bromobutyl)-3-methylisoxazole [VIII; R = $CH_3$, n = 4, Hal = Br] was prepared from 1,3-dibromopropane and 3,5-dimethylisoxazole according to the procedure of Example 9, part (c), and was obtained in 54% yield as a yellow oil after chromatography on silica gel.

b) 5-{4-[4-(4,5-Dihydro-2-oxazolyl)phenoxy]butyl}-3-methylisoxazole [I; R = $CH_3$, $R_1$, $R_2$, $R_3$, $R_4$ and $R_5$ = H, X = O, n = 4, oxazole at 4-position] was prepared from 5-(4-bromobutyl)-3-methylisoxazole and 4,5-dihydro-2-(4-hydroxyphenyl)oxazole according to the procedure of Example 9, part (d), and was obtained in 75% yield as a colorless solid, m.p. 93-94°C, when recrystallized from isopropyl acetate.

Example 22

5-{8-[2-Chloro-4-(4,5-dihydro-2-oxazolyl)phenoxy]octyl}-3-methylisoxazole [I; R = $CH_3$, $R_1$, $R_2$, $R_3$ and $R_4$ = H, $R_5$ = 2-Cl, X = O, n = 8, oxazole at 4-position] was prepared from 4,5-dihydro-2-(3-chloro-4-hydroxyphenyl)oxazole (Example 13b) and 5-(8-bromooctyl)-3-methylisoxazole (Example 18a) according to the procedure of Example 9, part (d), and was obtained in the form of a colorless solid, m.p. 63-64°C when recrystallized from ether.

Example 23

5-{7-[4-(4,5-Dihydro-5-hydroxymethyl-2-oxazolyl)phenoxy]heptyl}-3-methylisoxazole [I; R = $CH_3$, $R_1$, $R_2$, $R_4$ and $R_5$ = H, $R_3$ = $CH_2OH$, X = O, n = 7, oxazole at 4-position] was prepared from the ethyl imino-ester hydrochloride of 5-[7-(4-cyanophenoxy)heptyl]-3-methylisoxazole and 3-amino-1,2-propanediol according to the procedure of Example 1, part (c), and gas obtained in about 60% yield in the form of a colorless solid, m.p. 75-76°C, when recrystallized first from isopropyl acetate and then from acetonitrile.

Example 24

5-{4-[2-Chloro-4-(4,5-dihydro-2-oxazolyl)phenoxy]butyl]-3-methylisoxazole [I; R = $CH_3$, $R_1$, $R_2$, $R_3$ and $R_4$ = H, $R_5$ = 2-Cl, X = O, n = 4, oxazole at 4-position] was prepared from 4,5-dihydro-2-(3-chloro-4-hydroxyphenyl)oxazole (Example 13b) and 5-(4-bromobutyl)-3-methylisoxazole (Example 21a), according to the procedure of Example 9, part (d), and was obtained in 69% yield in the form of a colorless solid, m.p. 75-76°C, when recrystallized from isopropyl acetate.

Example 25

5-{7-[4-(4,5-Dihydro-4-hydroxymethyl-2-oxazolyl)phenyl]heptyl}-3-methylisoxazole [I; R = $CH_3$, $R_1$ = $CH_2OH$, $R_2$, $R_3$, $R_4$ and $R_5$ = H, X = single bond, n = 7, oxazole at 4-position] was prepared from the methyl imino-ester hydrochloride of 5-[7-(4-cyanophenyl)heptyl]-2-methylisoxazole and 2-amino-1,3-propanediol, according to the procedure of Example 6, part (g), and was obtained in about 40% yield in the form of a pale pink solid, m.p. 68-69°C, when recrystallized first from isopropyl acetatepentane and then from acetonitrile.

Example 26

a) 4,5-Dihydro-2-(3-hydroxyphenyl)oxazole [IX; $R_1'$, $R_2'$, $R_3'$ and $R_5$ = H, OH at 3-position] was prepared by reacting methyl 3-hydroxybenzoate and ethanolamine, and treating the resulting N-(2-hydroxyethyl)-3-hydroxybenzamide with thionyl chloride according to the procedures of Example 9, parts (a) and (b), and was obtained in 84% yield in the form of a colorless solid, m.p. 184-185°C, when recrystallized from an acetone—tetrahydrofuran mixture.

b) 5-{7-[3-(4,5-Dihydro-2-oxazolyl)phenoxy]heptyl}-3-methylisoxazole [I; R = $CH_3$, $R_1$, $R_2$, $R_3$, $R_4$ and $R_5$ = H, X = O, n = 7, oxazole at 3-position], was prepared from 4,5-dihydro-2-(3-hydroxyphenyl)-oxazole and 5-(7-bromoheptyl)-3-methylisoxazole according to the procedure of Example 9, part (d), and was obtained in 35% yield in the form of a colorless solid, m.p. 54-55 °C, when chromatographed and recrystallized from ether—hexane.

Example 27

a) 4,5-Dihydro-2-(3-chloro-4-hydroxyphenyl)-4-methyloxazole [IX; $R_1'$ = $CH_3$, $R_2'$ and $R_3'$ = H, $R_5$ = 3-Cl, OH at 4-position] was prepared by reacting methyl 3-chloro-4-hydroxybenzoate with 2-amino-1-propanol, and treating the resulting N-(1-methyl-2-hydroxyethyl)-3-chloro-4-hydroxybenzamidewith thionyl chloride according to the procedures of Example 9, parts (a) and (b), and was obtained in 69% yield as a colorless solid, m.p. 174-176 °C, when recrystallized from acetone.

b) 5-{8-[2-Chloro-4-(4,5-dihydro-4-methyl-2-oxazolyl)phenoxy]octyl}-3-methylisoxazole [I ; R and $R_1$ = $CH_3$, $R_2$, $R_3$ and $R_4$ = H, $R_5$ = 2-Cl, X = O, n = 8, oxazole at 4-position] was prepared by reacting 4,5-dihydro-2-(3-chloro-4-hydroxyphenyl)-4-methyloxazole with 5-(8-bromooctyl)-3-methylisoxazole (Example 18a) according to the procedure of Example 9, part (d), and was obtained in 67% yield in the form of a pale yellow oil, m.p. below room temperature.

Example 28

5-{7-[4-(4,5-Dihydro-2-oxazolyl)phenoxy]heptyl}-3-isoxazolemethanol acetate [I; R = $CH_3COOCH_2$, $R_1$, $R_2$, $R_3$, $R_4$ and $R_5$ = H, X = O, n = 7, oxazole at 4-position].

A solution of 2.80 g of 5-{7-[4-(4,5-dihydro-2-oxazolyl)phenoxy]heptyl}-3-isoxazolemethanol (Example 11) and 0.82 g of acetic anhydride in pyridine was allowed to stand overnight at room temperature. The reaction mixture was poured into ice water and the solid material which formed was collected, dried and recrystallized from isopropyl alcohol to give 2.5 g of 5-{7-[4-(4,5-dihydro-2-oxazolyl)phenoxy]heptyl}-3-isoxazolemethanol acetate, colorless solids m.p. 76-77 °C.

Example 29

a) 3,5-Dimethyl-4-hydroxymethylisoxazole.

To a suspension of 23.53 g of lithium aluminum hydride in 600 ml of ether was added dropwise 64 g of 4-carbethoxy-3,5-dimethylbisoxazole in 100 ml of ether at a rate so that gentle reflux occurred. The addition was complete in about two hours, and the mixture was stirred overnight under nitrogen. A saturated solution of sodium sulfate was added dropwise under nitrogen until the excess lithium aluminum hydride was decomposed. The resulting suspension was filtered and the filtrate concentrated to an oil which was distilled to give 36.4 g of 3,5-dimethyl-4-hydroxymethylisoxazole, b.p. 130-140 °C (0.1 mm).

b) 5-{7-[4-(4,5-Dihydro-2-oxazolyl)phenoxy]heptyl}-3-methyl-4-isoxazolemethanol [I; R = $CH_3$, $R_1$, $R_2$, $R_3$ and $R_5$ = H, $R_4$ = $CH_2OH$, X = O, n = 7, oxazole at 4-position] was prepared from 3,5-dimethyl-4-hydroxymethylisoxazole and 2-[4-(6-bromohexyloxy)phenyl]-4,5-dihydroxazole (Example 10c), according to the procedure described in Example 10, part (d), and was obtained in the form of a colorless solid, m.p. 95-97 °C, when recrystallized from acetonitrile.

Example 30

5-{5-[3-(4,5-Dihydro-2-oxazolyl)phenoxy]pentyl}-3-methylisoxazole [I; R = $CH_3$, $R_1$, $R_2$, $R_3$, $R_4$ and $R_5$ = H, X = O, n = 5, oxazole at 3-position] was prepared from 4,5-dihydro-2-(3-hydroxyphenyl)oxazole (Example 26a) and 5-(5-bromopentyl)-3-methylisoxazole (Example 15a) according to the procedure of Example 9, part (d), and was obtained in 49% yield in the form of colorless needles, m.p. 65-67 °C, when recrystallized from isopropyl acetate—hexane.

Example 31

a) 4,5-Dihydro-2-(4-hydroxy-3-methylphenyl)oxazole [IX; $R_1'$, $R_2'$ and $R_3'$ = H, $R_5$ = 3-$CH_3$, OH at 4-position] was prepared by reacting methyl 4-hydroxy-3-methylbenzoate and ethanolamine, and treating the resulting N-(2-hydroxyethyl)-4-hydroxy-3-methylbenzamide with thionyl chloride according to the procedures of Example 9, parts (a) and (b), and was obtained in 43% yield in the form of a colorless solid, m.p. 190-191 ° C, when recrystallized from methanol.

b) 5-{7-[4-(4,5-Dihydro-2-oxazolyl)-2-methylphenoxy]heptyl}-3-methylisoxazole [I; R = $CH_3$, $R_1$, $R_2$, $R_3$ and $R_4$ = H, $R_5$ = 2-$CH_3$, X = O, n = 7, oxazole at 4-position] was prepared from 4,5-dihydro-2-(4-hydroxy-3-methylphenyl)oxazole and 5-(7-bromoheptyl)-3-methylisoxazole according to the procedure of Example 9, part (d), and was obtained in 45% yield as a light-tan solid, m.p. 90-92 ° C, when recrystallized first from methanol and then from tertiary-butyl methyl ether.

Example 32

5-{5-[2-Chloro-4-(4,5-dihydro-4-methyl-2-oxazolyl)phenoxy]pentyl}-3-methylisoxazole [I; R and $R_1$ = $CH_3$, $R_2$, $R_3$ and $R_4$ = H, $R_5$ = 2-Cl, X = O, n = 5, oxazole at 4-position] was prepared from 3,5-dihydro-2-(3-chloro-4-hydroxyphenyl)-4-methyloxazole (Example 17a) and 5-(5-bromopentyl)-3-methylisoxazole (Example 15a) according to the procedure of Example 9, part (d), and was obtained in 51% yield in the form of a colorless solid, m.p. 81-83 ° C when recrystallized from a tertiary-butyl methyl ether—hexane mixture.

Example 33

5-{7-[4-(4,5-Dihydro-4-hydroxymethyl-2-oxazolyl)phenoxylheptyl}-3-methylisoxazole [I; R = $CH_3$, $R_1$ = $CH_2OH$, $R_2$, $R_3$, $R_4$ and $R_5$ = H, X = O, n = 7, oxazole at 4-position] was prepared from the ethyl imino-ester hydrochloride of 5-[7-(4-cyanophenoxy)heptyl]-2-methylisoxazole and 2-amino-1,3-propanediol, according to the procedure of Example 1, part (c), and was obtained in 73% yield in the form of a colorless solid, m.p. 77-78 ° C when recrystallized from an isopropyl acetate—hexane mixture.

Example 34

a) 4,5-Dihydro-2-(4-hydroxyphenyl)-5-methyloxazole [IX; $R_1'$, $R_2'$, $R_5$ = H, $R_3'$ = $CH_3$, OH at 4-position] was prepared by reacting methyl 4-hydroxybenzoate with 1-amino-2-propanol and treating the resulting N-(2-hydroxypropyl)-4-hydroxybenzamide with thionyl chloride according to the procedures of Example 9, parts (a) and (b) and was obtained in 29% yield in the form of a colorless solid, m.p. 207-209 ° C, when recrystallized from an acetonitrile— tetrahydrofuran mixture.

b) 5-{7-[4-(4,5-Dihydro-5-methyl-2-oxazolyl)phenoxy]heptyl}-3-methylisoxazole [I; R and $R_3$ = $CH_3$, $R_1$, $R_2$, $R_4$ and $R_5$ = H, X = O, n = 7, oxazole at 4-position] was prepared from 4,5-dihydro-2-(4-hydroxyphenyl)-5-methyloxazole and 5-(7-bromopentyl)-3-methylisoxazole according to the procedure of Example 9, part (d), and was obtained in the form of a colorless solid, m.p. 81-82 ° C when recrystallized from acetonitrile.

Example 35

4-Chloromethyl-5-{7-[4-(4,5-dihydro-2-oxazolyl)phenoxyl]heptyl}-3-methylisoxazole [I; R = $CH_3$, $R_1$, $R_2$, $R_3$ and $R_5$ = H, $R_4$ = $CH_2Cl$, X = O, n = 7, oxazole at 4-position].

To a solution of 2.1 ml of thionyl chloride in 5.0 ml of methylene dichloride cooled in an ice-bath was added over a 30 minute period a suspension of 5 g of 5-{7-[4-(4,5-dihydro-2-oxazolyl)phenoxy]heptyl}-3-methyl-4-isoxazolemethanol (Example 29b) in 20 ml of methylene dichloride. The reaction mixture was stirred at 0 ° C for 2 hours and then at room temperature for 3 hours. The solvent was then removed in vacuo and the residue triturated with ether. The solid product was collected and recrystallized from acetonitrile to give 4.5 g of 4-chloromethyl-5-{7-[4-(4,5-dihydro-2-oxazolyl)phenoxy]heptyl}-3-methylisoxazole in the form of its monohydrochloride salt, tan solid, m.p. 103-104 ° C.

Example 36

5-{7-[4-(4,5-Dihydro-2-oxazolyl)phenoxy]heptyl}-3-methyl-4-(1-pyrrolidylmethyl)isoxazole [I; R = $CH_3$, $R_1$, $R_2$, $R_3$ and $R_5$ = H, $R_4$ = 1-pyrrolidylmethyl, X = O, n = 7, oxazole at 4-position].

A solution of 2.8 g of 4-chloromethyl-5-{7-[4-(4,5-dihydro-2-oxazolyl)phenoxy]heptyl}-3-methylisoxazole (Example 35) and 1.3 g of pyrrolidine in 50 ml of dimethylformamide was heated on a steam bath for six hours and then kept overnight at room temperature. The solvent was removed in vacuo, and the residue was dissolved in water and made basic with sodium bicarbonate solution. The solid product which separated was collected and dried to give 1.3 g of 5-{7-[4-(4,5-dihydro-2-oxazolyl)phenoxy]heptyl}-3-methyl-4-(1-pyrrolidylmethyl)isoxazole, m.p. 98-99 ° C.

By replacing the pyrrolidine in the foregoing example by a molar equivalent amount of ammonia, ethylamine, dimethylamine, piperidine or morpholine, it is contemplated that there can be obtained, respectively, 4-aminomethyl-5-{7-[4-(4,5-dihydro-2-oxazolyl)phenoxy]heptyl}-3-methylisoxazole; 5-{7-[4-(4,5-dihydro-2-oxazolyl)phenoxy]heptyl}-4-ethylaminomethyl-3-methylisoxazole; 5-{7-[4-(4,5-dihydro-2-oxazolyl)phenoxy]heptyl}-4-dimethylaminomethyl-3-methylisoxazole; 5-{7-[4-(4,5-dihydro-2-oxazolyl)phenoxy]-heptyl}-3-methyl-4-(1-piperidylmethyl)isoxazole; or 5-{7-[4-(4,5-dihydro-2-oxazolyl)phenoxy]heptyl}-3-methyl-4-(4-morpholinylmethyl)isoxazole.

It is further contemplated that the 4-aminomethyl-5-{7-[4-(4,5-dihydro-2-oxazolyl)phenoxy]heptyl}-3-methylisoxazole can be caused to react with acetyl chloride to produce the corresponding 4-acetylamino compound.

Example 37

3-{5-[2-Chloro-4-(4,5-dihydro-2-oxazolyl)phenoxy]pentyl}-5-methylisoxazole [II; $R_1$, $R_2$ and $R_3$ = H, $R_5$ = 2-Cl, $R_6$ = $CH_3$, n = 5, oxazole at 4-position] was prepared from 3-(5-bromopentyl)-5-methylisoxazole (Example 7f) and 4,5-dihydro-2-(3-chloro-4-hydroxyphenyl)oxazole (Example 13b) according to the procedure of Example 9, part (d), and was obtained in 62% yield in the form of a colorless solid, m.p. 102-103 ° C when recrystallized from methanol.

Example 38

a) 4,5-Dihydro-2-(4-hydroxy-2-methylphenyl)oxazole [IX; $R_1'$, $R_2'$ and $R_3'$ = H, $R_5$ = 2-$CH_3$, OH at 4-position] was prepared by reacting methyl 4-hydroxy-2-methylbenzoate and ethanolamine, and treating the resulting N-(2-hydroxyethyl)-4-hydroxy-2-methylbenzamide with thionyl chloride according to the procedures of Example 9, parts (a) and (b), and was obtained in 34% yield, m.p. 147-149 ° C, when recrystallized first from acetonitrile and then from methanol.

b) 5-{7-[4-(4,5-Dihydro-2-oxazolyl)-3-methylphenoxy]heptyl}-3-methylisoxazole [I; R = $CH_3$, $R_1$, $R_2$, $R_3$ and $R_4$ = H, $R_5$ = 3-$CH_3$, X = O, n = 7, oxazole at 4-position] was prepared from 4,5-dihydro-2-(4-hydroxy-2-methylphenyl)oxazole and 5-(7-bromoheptyl)-3-methylisoxazole according to the procedure of Example 9, part (d) and was obtained in 27% yield as a colorless solid, m.p. 58-59 ° C, when recrystallized from an isopropyl acetate—hexane mixture.

Example 39

5-{7-[4-(5-Chloromethyl-4,5-dihydro-2-oxazolyl)phenoxy]heptyl}-3-methylisoxazole [I; R = $CH_3$, $R_1$, $R_2$, $R_4$ and $R_5$ = H, $R_3$ = $CH_2Cl$, X = O, n = 7, oxazole at 4-position] was prepared from 5-{7-[4-(4,5-dihydro-5-hydroxymethyl-2-oxazolyl)phenoxy]heptyl}-3-methylisoxazole (Example 23) and thionyl chloride, according to the procedure of Example 35, and was obtained in the form of a tan solid, m.p. 107-109 ° C, when recrystallized from ethyl acetate.

Example 40

5-{7-[4-(4,5-Dihydro-5-methoxymethyl-2-oxazolyl)phenoxy]heptyl}-3-methylisoxazole [I; R = $CH_3$, $R_1$, $R_2$, $R_4$ and $R_5$ = H, $R_3$ = $CH_2OCH_3$, X = O, n = 7].

To a stirred suspension of 1.08 g of sodium hydride (60% in mineral oil) in 50 ml of dry tetrahydrofuran was added over 33 minutes a solution of 6.6 g of 5-{7-[4-(4,5-dihydro-5-hydroxymethyl-2-oxazolyl)phenoxy]-heptyl}-3-methylisoxazole (Example 23). The mixture was heated at gentle reflux for one hour, then cooled to room temperature, and 4.30 g of methyl iodide in 25 ml of dry tetrahydrofuran was added over a 15 minute period. The reaction mixture was stirred overnight at room temperature, then filtered and concentrated in vacuo. The residue was washed with n-pentane and dissolved in ethyl acetate and the soluble portion isolated (6.31 g, m.p. 53-55 ° C). The latter was recrystallized from hexane to give 5.1 g of 5-{7-[4-(4,5-dihydro-5-methoxymethyl-2-oxazolyl)phenoxy]heptyl}-3-methylisoxazole, m.p. 60-61 ° C.

Example 41

a) 4,5-Dihydro-2-(4-hydroxy-3-methoxyphenyl)oxazole [IX; $R_1'$, $R_2'$, $R_3'$ = H, $R_5$ = 3-$OCH_3$, OH at 4-position] was prepared by reacting methyl 4-hydroxy-3-methoxybenzoate and ethanolamine, and treating the resulting N-(2-hydroxyethyl)-4-hydroxy-3-methoxybenzamide with thionyl chloride according to the procedures of Example 9, parts (a) and (b), and was obtained in 48% yield in the form of a colorless solid, m.p. 184-185 °C when recrystallized from methanol.

b) 5-{7-[4-(4,5-Dihydro-2-oxazolyl)-2-methoxyphenoxy]heptyl}-3-methylisoxazole [I; R = $CH_3$, $R_1$, $R_2$, $R_3$ and $R_4$ = H, $R_5$ = 2-$OCH_3$, X = O, n = 7, oxazole at 4-position] was prepared from 4,5-dihydro-2-(4-hydroxy-3-methoxyphenyl)oxazole and 5-(7-bromoheptyl)-3-methylisoxazole according to the procedure of Example 9, part (d), and was obtained in the form of colorless crystals, m.p. 71-73 °C when recrystallized from a hexane—isopropyl acetate mixture.

Example 42

5-{5-[4-(4,5-Dihydro-2-oxazolyl)-2-methoxyphenoxy]pentyl}-3-methylisoxazole [I; R = $CH_3$, $R_1$, $R_2$, $R_3$ and $R_4$ = H, $R_5$ = 2-$OCH_3$, X = O, n = 5, oxazole at 4-position] was prepared from 4,5-dihydro-2-(4-hydroxy-3-methoxyphenyl)oxazole (Example 41a) and 5-(5-bromopentyl)-3-methylisoxazole according to the procedure of Example 9, part (d), and was obtained in the form of a colorless solid, m.p. 97-99 °C. when recrystallized from isopropyl acetate.

It is further contemplated that by carrying out the procedures of Example 9 but replacing the methyl 4-hydroxybenzoate in part (a) of that example by a molar equivalent amount of methyl 3-nitro-4-hydroxybenzoate, methyl 3-methylthio-4-hydroxybenzoate, or methyl 3-trifluoromethyl-4-hydroxybenzoate, there can be obtained, respectively, 5-[7-[4-(4,5-dihydro-2-oxazolyl)-2-nitrophenoxy]heptyl}-3-methylisoxazole [I; R = $CH_3$, $R_1$, $R_2$, $R_3$ and $R_4$ = H, $R_5$ = 2-$NO_2$, X = O, n = 7, oxazole at 4-position]; 5-{7-[4-(4,5-dihydro-2-oxazolyl)-2-methylthiophenoxy]heptyl}-3-methylisoxazole [I; R = $CH_3$, $R_1$, $R_2$, $R_3$ and $R_4$ = H, $R_5$ = 2-$SCH_3$, X = O, n = 7, oxazole at 4-position]; or 5-{7-[4-(4,5-dihydro-2-oxazolyl)-2-trifluoromethylphenoxy]-heptyl}-3-methylisoxazole [I; R = $CH_3$, $R_1$, $R_2$, $R_3$ and $R_4$ = H, $R_5$ = 2-$CF_3$, X = O, n = 7, oxazole at 4-position].

Biological evaluation of compounds of the invention has shown that they possess antiviral activity. They are useful in inhibiting virus replication in vitro as well as in animals. The in vitro testing of the compounds of the invention against picornaviruses showed that viral growth was inhibited at minimim inhibitory concentrations (MIC) ranging from about 0.003 to about 1.5 micrograms per milliliter.

The MIC values were determined by a standard plaque reduction assay as follows: HeLa (Ohio) cells in monolayers were infected at a concentration of virus to give approximately 80 plaques per monolayer in the virus control (no drug present). The compound to be tested was serially diluted and included during infection and in the agar-medium overlay. The MIC was determined to be that concentration of compound which reduced the number of plaques by 50% with respect to the untreated virus control.

Extensive testing of a preferred compound, 5-{7-[4-(4,5-dihydro-2-oxazolyl)phenoxy]heptyl}-3-methylisoxazole, demonstrated in vitro activity against a variety of picornaviruses, including numerous strains of rhinoviruses and enteroviruses, as well as in vivo activity against poliovirus infections in mice.

In the in vivo studies, white Swiss mice averaging 20 g in weight were infected with 2 $LD_{50}$'s of poliovirus, type 2, MEF strain, by injecting 0.03 ml of the virus (540 pfu) into the left cerebral hemisphere. The mice were medicated intragastrically with the test compound as a suspension in gum tragacanth one hour prior to infection, 6 hrs post-infection and then b.i.d. for a total of 10 days. Appropriate placebo-medicated mice were included in the test, and all mice were checked twice daily for deaths. The test was terminated at 14 days post-infection.

The following tables give the results obtained with the compounds of the invention.

TABLE I

*In vitro* MICs (μg/ml) vs Selected Picornaviruses

| Example No. | Enteroviruses | | | | | Rhinoviruses | | | | | | |
|---|---|---|---|---|---|---|---|---|---|---|---|---|
| | Polio-I | Polio-II | Polio-III | ECHO-9 | ECHO-11 | 1A | 1B | 2 | 16 | 18 | 21 | 22 |
| 1(c) | 0.08 | 0.004 | 0.006 | 0.01 | 0.04 | 1.0 | 1.0 | 0.08 | 0.03 | 0.69 | 0.17 | 0.12 |
| 2 | 0.08 | 0.003 | 0.003 | 0.1 | 0.02 | NT* | NT | 0.1 | NT | NT | NT | NT |
| 3 | NT | 0.12 | NT | NT | NT | 1.5 | 1.2 | 0.04 | 0.08 | 0.4 | 0.1 | 0.02 |
| 4 | 0.4 | 0.16 | 0.03 | 0.006 | 0.02 | NT | NT | 0.02 | NT | NT | NT | NT |
| 5 | NT | 1.1 | NT | NT | NT | NT | NT | 0.06 | NT | NT | NT | NT |
| 6(g) | NT | 0.09 | NT | NT | NT | NT | NT | 0.06 | NT | NT | NT | NT |
| 7(k) | 0.2 | 0.005 | 0.002 | NT | 0.005 | NT | NT | 0.1 | NT | NT | NT | NT |
| 11 | 0.3 | 0.02 | 0.005 | NT | NT | NT | NT | 0.3 | NT | NT | NT | NT |
| 12 | NT | 0.08 | NT | NT | NT | NT | NT | 1.4 | NT | NT | NT | NT |
| 13(c) | 0.04 | 0.04 | 0.015 | 0.01 | 0.003 | 0.6 | 0.2 | 0.03 | 0.04 | NT | 0.09 | 0.05 |
| 14(b) | NT | 0.008 | NT | NT | NT | NT | NT | 0.12 | NT | NT | NT | NT |
| 15(b) | 0.09 | 0.05 | 0.04 | 0.05 | 0.002 | 0.16 | 0.05 | 0.004 | 0.025 | NT | 0.01 | 0.04 |

*Not tested

TABLE II

| In vitro MICs (μg/ml) | | |
|---|---|---|
| Example No. | Polio II | Rhinovirus 2 |
| 16 | 0.02 | 0.012 |
| 17(b) | 0.4 | 0.06 |
| 18(b) | 0.02 | 0.16 |
| 19(b) | 0.03 | 0.04 |
| 20 | 0.01 | 0.01 |
| 21(b) | 0.5 | 0.5 |
| 22 | 1.1* | <0.4* |
| 23 | NA** | 2.9* |
| 24 | 0.55 | 0.02 |
| 25 | NA** | 2.1* |
| 26(b) | NA** | 0.9* |
| 27(b) | >0.2 | 0.02 |
| 28 | 0.007 | 0.1 |
| 29(b) | 0.004 | 0.2 |
| 30 | >0.2 | 0.08 |
| 31(b) | 0.04 | 0.008 |
| 32 | 0.2 | <0.01 |
| 33 | >0.2 | 0.2 |
| 34(b) | 0.05 | <0.01 |
| 35 | <0.005 | 0.2 |

*compound present in overlay only
**compound present in overlay only (NA = inactive)

TABLE III

| In vivo vs Poliovirus-2 | |
|---|---|
| Example No. | Survivors Day 14 Post Infection |
| 1(c) | 50 mg/kg/day 14/20<br>100 mg/kg/day - 18/20<br>placebo - 1/20 |
| 2 | 77 mg/kg/day - 17/20<br>placebo - 6/20 |
| 3 | 100 mg/kg/day - 9/20<br>200 mg/kg/day - 12/20<br>400 mg/kg/day - 14/20<br>placebo - 6/20 |
| 6(g) | 50 mg/kg/day - 5/20<br>100 mg/kg/day - 17/20<br>200 mg/kg/day - 19/20<br>placebo - 1/20 |
| 7(k) | 100 mg/kg/day - 16/20<br>placebo - 6/20 |
| 13(c) | 25 mg/kg/day - 4/19<br>50 mg/kg/day - 11/17<br>100 mg/kg/day - 16/20<br>placebo - 2/17 |
| 15(b) | 50 mg/kg/day - 7/20<br>100 mg/kg/day - 18/20<br>placebo - 4/20 |

The antiviral compositions ore formulated for use by preparing a dilute solution or suspension in a pharmaceutically acceptable aqueous, organic or aqueous-organic medium for parenteral administration by intravenous or intramuscular injection, or for intranasal or ophthalmic application; or are prepared in tablet or capsule form with conventional excipients for oral administration.

## Claims

### Claims for the following Contracting States : BE, CH, DE, FR, GB, IT, LI, NL, SE

**1.** A compound having the formula

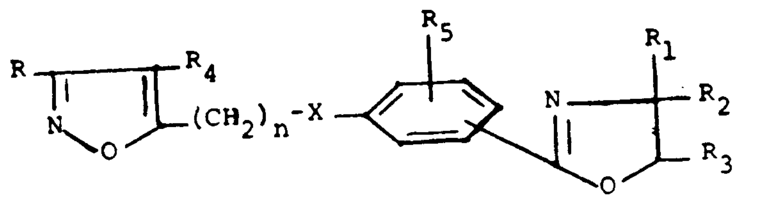

...I

or

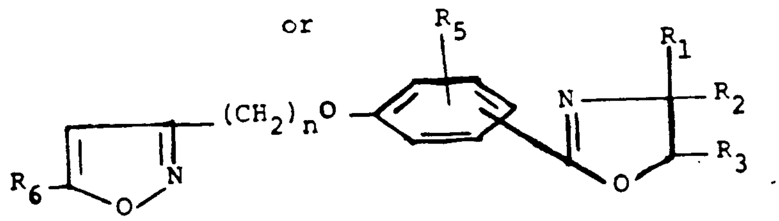

...II

wherein:

R, $R_1$, $R_2$, $R_3$ and $R_4$ are each hydrogen or alkyl of 1 to 3 carbon atoms optionally substituted by hydroxy, lower-alkanoyloxy of from 1 to 4 carbon atoms, lower-alkoxy of from 1 to 4 carbon atoms, chloro, or N=Z, wherein N=Z is amino, lower-alkanoylamino of from 1 to 4 carbon atoms, 1-pyrrolidinyl, 1-piperidinyl or 4-morpholinyl; with the proviso that R is other than hydrogen;

$R_5$ is hydrogen, lower-alkyl of 1-4 carbon atoms, halogen, nitro, lower-alkoxy of 1-4 carbon atoms, lower-alkylthio of 1-4 carbon atoms or trifluoromethyl;

$R_6$ is alkyl of 1 to 3 carbon atoms;

X is O or a single bond; and

n is an integer from 3 to 9;

or a pharmaceutically accetable acid-addition salt thereof.

**2.** A compound according to claim 1: 5-{7-[4-(4,5-dihydro-2-oxazolyl)phenoxy]heptyl}-3-methylisoxazole, or a pharmaceutically acceptable acid-addition salt thereof.

**3.** A compound according to claim 1: 5-{7-[2-chloro-4-(4,5-dihydro-2-oxazolyl)phenoxy]heptyl}-3-methylisoxazole, or a pharmaceutically acceptable acid-addition salt thereof.

**4.** A compound according to claim 1: 5-{5-[2-chloro-4-(4,5-dihydro-2-oxazolyl)phenoxy]pentyl}-3-methylisoxazole, or a pharmaceutically acceptable acid-addition salt thereof.

**5.** A compound having the formula

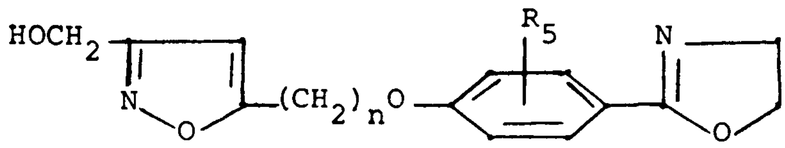

III

or

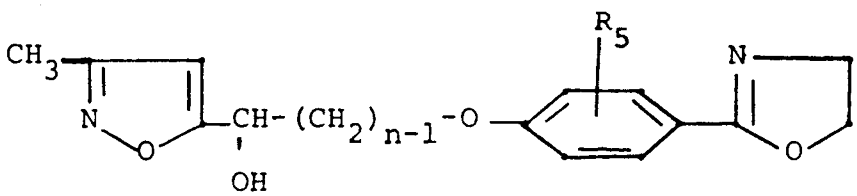

IV

wherein $R_5$ is hydrogen, lower-alkyl of 1 to 4 carbon atoms, halogen, nitro, lower-alkoxy of 1-4 carbon atoms, lower-alkylthio of 1-4 carbon atoms or trifluoromethyl and n is an integer from 3 to 9, or a pharmaceutically acceptable acid-addition salt thereof.

**6.** A process for preparing a compound according to claim 1, which comprises:

a. reacting a compound of the formula

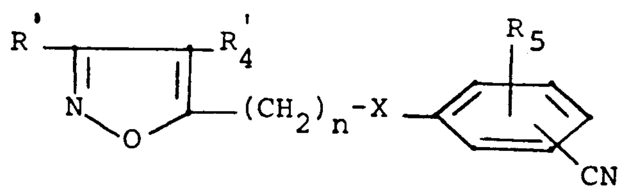

...V

or

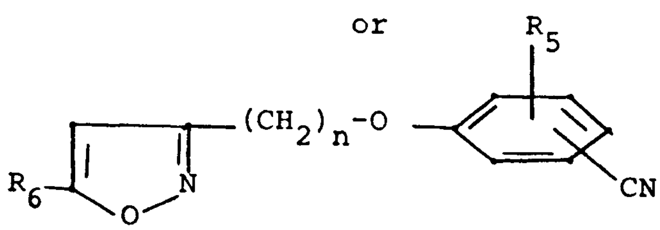

...VI

with a lower-alkanol in the presence of a strong acid, and heating the resulting imino ester with a compound of the formula

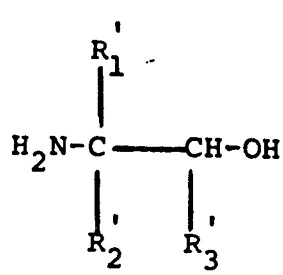

wherein R' is alkyl or hydroxyalkyl of 1 to 3 carbon atoms, and $R_1'$, $R_2'$, $R_3'$ and $R_4'$ are each hydrogen, or alkyl or hydroxyalkyl of 1 to 3 carbon atoms, so as to prepare the corresponding compound of Formula I or II or

b. reacting a compound of the formula

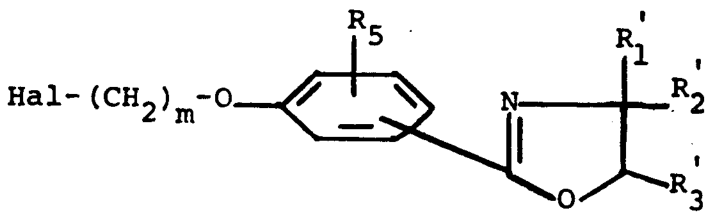

...VII

wherein Hal is bromine or iodine, and m = n-1, with an alkali metal derivative of 3-R'-4-$R_4'$ -5-methylisoxazole; wherein R' is alkyl or hydroxyalkyl of 1 to 3 carbon atoms; $R_2'$, $R_2'$, $R_3'$ and $R_4'$ are each hydrogen, or alkyl or hydroxyalkyl of 1 to 3 carbon atoms so as to prepare the corresponding compound of Formula I where X is O or

c. reacting a compound of the formula

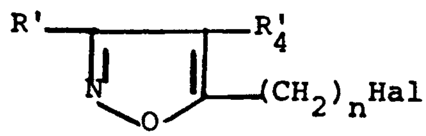

with an alkali metal salt of a compound of the formula

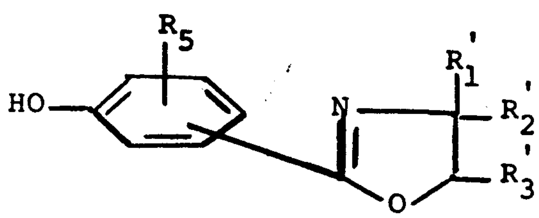

...IX

wherein R' is alkyl or hydroxyalkyl of 1 to 3 carbon atoms; $R_1'$, $R_2'$, $R_3'$ and $R_4'$ are each hydrogen, or alkyl or hydroxyalkyl of 1 to 3 carbon atoms so as to prepare the corresponding compound of Formula I where X is O,

if desired, converting a compound of Formula I or II obtained where one or more of R, $R_1$, $R_2$, $R_3$ or $R_4$ is hydroxyalkyl as follows:

d. esterifying said compound to prepare the corresponding compound where said one or more radicals is lower-alkanoyloxyalkyl,

e. etherifying said compound to prepare the corresponding compound were said one or more radicals is lower alkoxyalkyl,

f. subjecting said compound to the action of a reagent capable of replacing aliphatic hydroxyl groups by chlorine to prepare the corresponding compound where said one or more radicals is chloroalkyl,

g. subjecting a compound thus obtained where one or more of R, $R_1$, $R_2$, $R_3$ or $R_4$ is chloroalkyl to reaction with ammonia or an amine, HN=Z, to prepare the corresponding compound where said radical or radicals is alkyl-N=Z, wherein N=Z is amino, lower-alkylamino, di-lower-alkylamino, 1-pyrrolidinyl, 1-piperidinyl or 4-morpholinyl,

h. reacting a compound thus obtained where said one or more radicals is aminoalkyl with a lower-alkanoyl halide or anhydride to prepare the corresponding compound where said one or more radicals is lower-alkanoylaminoalkyl,

and, if desired, converting a free base obtained to a pharmaceutically acceptable acid addition salt thereof.

**7.** A process for preparing a compound according to claim 5, which comprises subjecting to suitable fermentative enzymatic action, for example using Aspergillus niger ($A_1$) or Trichotecium roseum ($T_1$), a compound of Formula I according to claim 1, wherein R is $CH_3$, $R_1$, $R_2$, $R_3$ and $R_4$ are H, X is O, the dihydro-oxazole moiety is in the 4-position of the phenylene ring,

and, if desired, converting a free base obtained to a pharmaceutically acceptable acid-addition salt thereof.

**8.** A compound having the formula IX

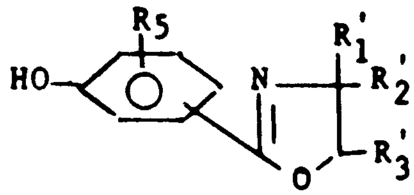

wherein the hydroxy group is in a position para to the oxazoline group and/or VII

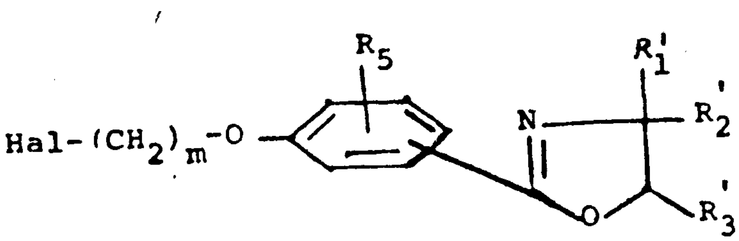

wherein $R_1'$, $R_2'$ and $R_3'$ are each hydrogen, or alkyl or hydroxyalkyl of 1 to 3 carbon atoms; $R_5$ is hydrogen, lower-alkyl of 1-4 carbon atoms, halogen, nitro, lower-alkoxy of 1-4 carbon atoms, lower-alkylthio of 1-4 carbon atoms or trifluoromethyl; Hal is bromine or iodine; and m is an integer from 2 to 8 provided that not all $R_1'$, $R_2'$, $R_3'$ and $R_5$ are hydrogen.

**9.** A process for preparing a compound according to claim 8, which comprises cyclizing a compound of the formula

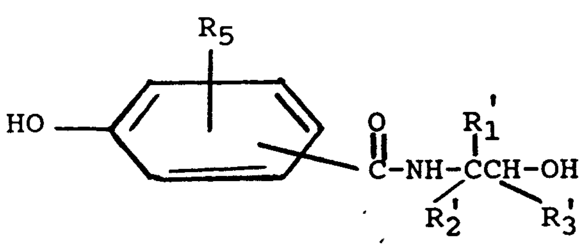

...XVII

to prepare a compound of the Formula IX, if desired, etherifying a compound of the Formula IX obtained with an alkylene dibromide to obtain a compound of the Formula VII where Hal is bromine, and, if desired, replacing the bromide with an iodide to obtain the compound of Formula VII where Hal is iodine.

**10.** A composition for combatting viruses which comprises an antivirally effective amount of a compound according to any one of claims 1-5 in admixture with a suitable carrier or diluent.

**Claims for the following Contracting State : AT**

**1.** A process for preparing a compound having the formula

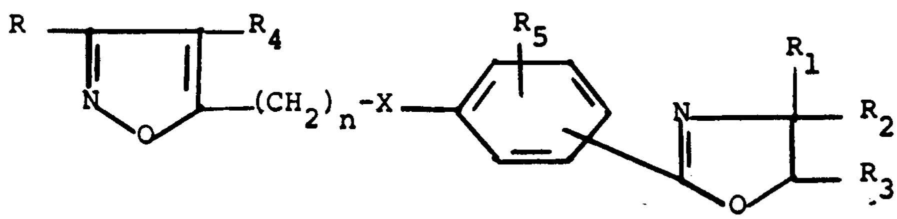

...I

or

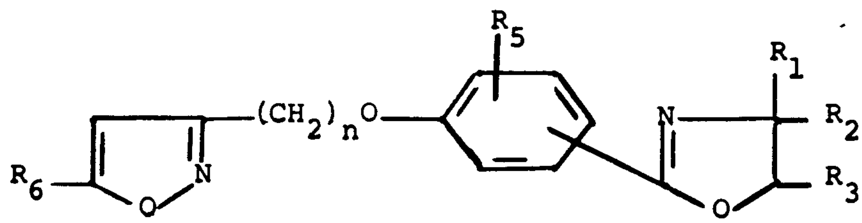

...II

wherein:

R, $R_1$, $R_2$, $R_3$ and $R_4$ are each hydrogen or alkyl of 1 to 3 carbon atoms optionally substituted by hydroxy, lower-alkanoyloxy, lower-alkoxy, chloro, or N = Z, wherein N = Z is amino, lower-alkanoylamino, 1-pyrrolidinyl, 1-piperidinyl or 4-morpholinyl; with the proviso that R is other than hydrogen;

$R_5$ is hydrogen, lower-alkyl of 1-4 carbon atoms, halogen, nitro, lower-alkoxy of 1-4 carbon atoms, lower-alkylthio of 1-4 carbon atoms or trifluoromethyl;

$R_6$ is alkyl of 1 to 3 carbon atoms;

X is O or a single bond; and

n is an integer from 3 to 9;

or a pharmaceutically accetable acid-addition salt thereof, characterized by:

a. reacting a compound of the formula

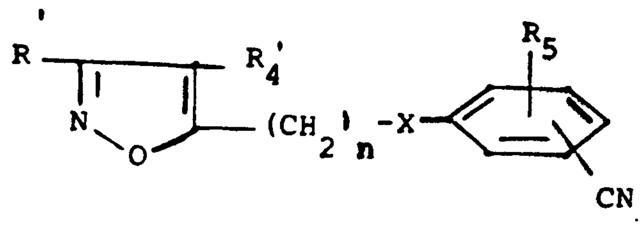

...V

or

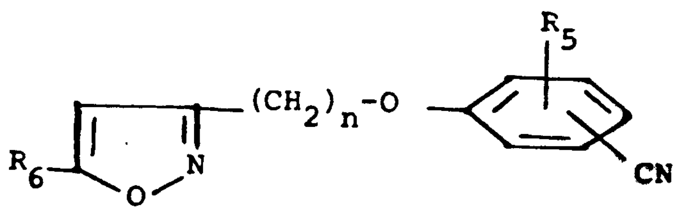

...VI

with a lower-alkanol in the presence of a strong acid, and heating the resulting imino ester with a compound of the formula

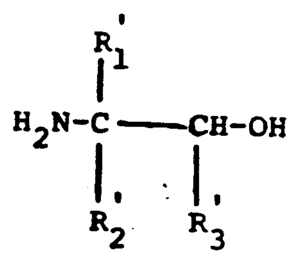

wherein R' is alkyl or hydroxyalkyl of 1 to 3 carbon atoms, and $R_1'$ , $R_2'$ , $R_3'$ and $R_4'$ are each hydrogen, or alkyl or hydroxyalkyl of 1 to 3 carbon atoms, so as to prepare the corresponding compound of Formula I or II or

b. reacting a compound of the formula

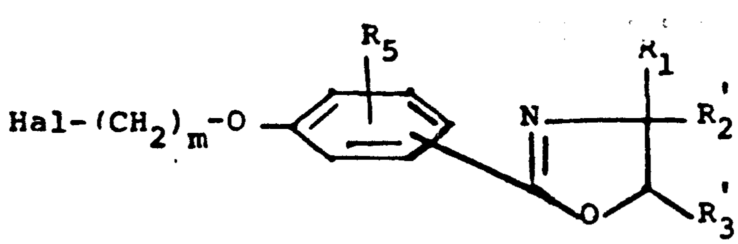

...VII

wherein Hal is bromine or iodine, and m = n-1, with an alkali metal derivative of 3-R'-4-$R_4'$ -5-methylisoxazole; wherein R' is alkyl, or hydroxyalkyl of 1 to 3 carbon atoms; $R_1'$ , $R_2'$ , $R_3'$ and $R_4'$ are each hydrogen, or alkyl or hydroxyalkyl of 1 to 3 carbon atoms so as to prepare the corresponding compound of Formula I where X is O or

c. reacting a compound of the formula

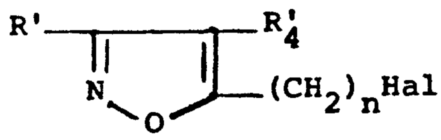

with an alkali metal salt of a compound of the formula

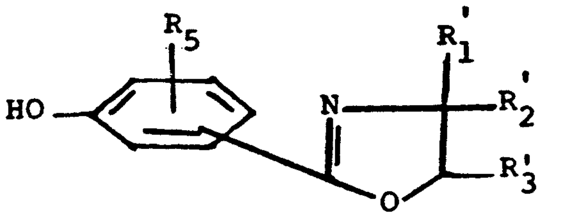

...IX

wherein R' is alkyl or hydroxyalkyl of 1 to 3 carbon atoms: $R_1'$, $R_2'$, $R_3'$ and $R_4'$ are each hydrogen, or alkyl or hydroxyalkyl of 1 to 3 carbon atoms so as to prepare the corresponding compound of Formula I where X is O,

if desired, converting a compound of Formula I or II obtained where one or more of R, $R_1$, $R_2$, $R_3$ or $R_4$ is hydroxyalkyl as follows:

d. esterifying said compound to prepare the corresponding compound where said one or more radicals is lower-alkanoyloxyalkyl,

e. etherifying said compound to prepare the corresponding compound where said one or more radicals is lower-alkoxyalkyl,

f. subjecting said compound to the action of a reagent capable of replacing aliphatic hydroxyl groups by chlorine to prepare the corresponding compound where said one or more radicals is chloroalkyl,

g. subjecting a compound thus obtained where one or more of R, $R_1$, $R_2$, $R_3$ or $R_4$ is chloroalkyl to reaction with ammonia or an amine, HN = Z, to prepare the corresponding compound where said radical or radicals is alkyl-N = Z, wherein N = Z is amino, lower-alkylamino, di-lower-alkylamino, 1-pyrrolidinyl, 1-piperidinyl or 4-morpholinyl,

h. reacting a compound thus obtained where said one or more radicals is aminoalkyl with a lower-alkanoyl halide or anhydride to prepare the corresponding compound where said one or more radicals is lower-alkanoylaminoalkyl,

and, if desired, converting a free base obtained to a pharmaceutically acceptable acid addition salt thereof.

**2.** A process according to claim 1, wherein 5-{7-[4-(4,5-dihydro-2-oxazolyl)phenoxy]heptyl}-3-methylisoxazole is prepared by reacting 5-[7-(4-cyanophenoxy)-heptyl]-3-methylisoxazole with a lower-alkanol in the presence of a strong acid, and heating the resulting imino ester with 2-hydroxyethylamine.

**3.** A process according to claim 1, wherein 6-[4-(4,5-dihydro-2-oxazolyl)phenoxy]hexyl halide is reacted with an alkali metal derivative of 3,5-dimethylisoxazole to produce 5-{7-[4-(4,5-dihydro-2-oxazolyl)-phenoxy]heptyl}-3-methylisoxazole.

**4.** A process according to claim 1, wherein 5-(6-halohexyl)-3-methylisoxazole is reacted with an alkali metal salt of 2-(4-hydroxyphenyl)-4,5-dihydrooxazole to produce 5-{7-[4-(4,5-dihydro-2-oxazolyl)-phenoxy]heptyl}-3-methylisoxazole.

**5.** A process for preparing a compound having the formula

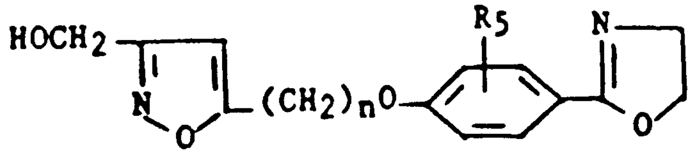

III

or

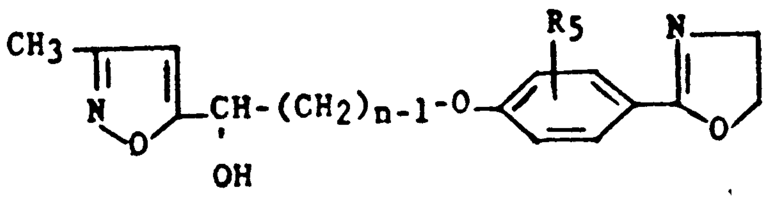

IV

,

wherein $R_5$ is hydrogen, lower-alkyl of 1 to 4 carbon atoms, halogen, nitro, lower-alkoxy of 1 to 4 carbon atoms, lower-alkylthio of 1 to 4 carbon atoms or trifluoromethyl and n is an integer from 3 to 9, or a pharmaceutically acceptable acid-addition salt thereof, characterized by subjecting to suitable fermentative enzymatic action a compound of Formula I as defined in claim 1, wherein R is $CH_3$, $R_1$, $R_2$, $R_3$ and $R_4$ are H, X is O the dihydro-oxazole moiety is in the 4-position of the phenylene ring,

and, if desired, converting a free base obtained to a pharmaceutically acceptable acid-addition salt thereof.

**6.** A process according to claim 5, in which $R_5$ is hydrogen and n is 7 is produced using Aspergillus niger ($A_1$) or Trichotecium roseum ($T_1$).

**7.** A process for preparing a compound having the formula IX or VII wherein $R'_1$, $R'_2$, and $R'_3$ are each hydrogen, or alkyl or hydroxyalkyl of 1 to 3 carbon atoms; $R_5$ is hydrogen, lower-alkyl of 1 to 4 carbon atoms, halogen, nitro, lower-alkoxy of 1 to 4 carbon atoms, lower-alkylthio of 1 to 4 carbon atoms or trifluoromethyl; Hal is bromine or iodine; and m is an integer from 2 to 8, characterized by cyclizing a compound of the formula

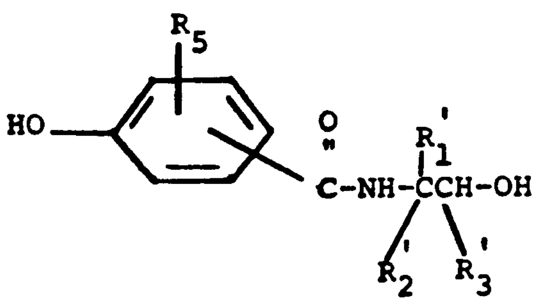

...XVII

to prepare a compound of the Formula IX, if desired, etherifying a compound of the Formula IX obtained with an alkylene dibromide to obtain a compound of the Formula VII where Hal is bromine, and, if desired, replacing the bromide with an iodide to obtain the compound of Formula VII where Hal is iodine.

**8.** A process according to any one of claims 1, 5 and 7, in which the starting compounds are chosen so that in the compound of Formula I produced R and $R_6$ are each alkyl of 1-3 carbon atoms, $R_1$ and $R_2$ are each hydrogen, or alkyl or hydroxyalkyl of 1 to 3 carbon atoms, $R_3$, $R_4$ and $R_5$ are each hydrogen,

n is an integer from 4 to 8 when separated by O from the phenylene ring or an integer from 5 to 9 in Formula I when X is a single bond, and the dihydro-oxazole moiety is affixed to the 4-position of the phenylene ring.

**9.** A process according to any one of claims 1, 5 and 7, in which the starting compounds are chosen so that in the compound of Formula I produced a compound according to claim 8 is excluded.

**Revendications**

**Revendications pour les Etats contractants suivants : BE, CH, DE, FR, GB, IT, NL, SE**

**1.** Un composé répondant à l'une des formules :

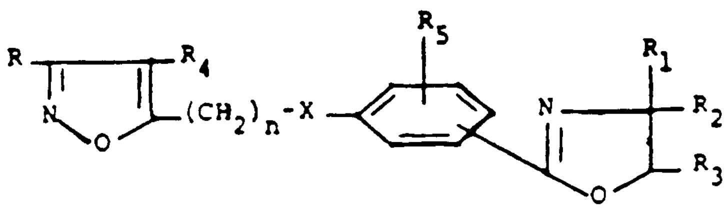

...I

et

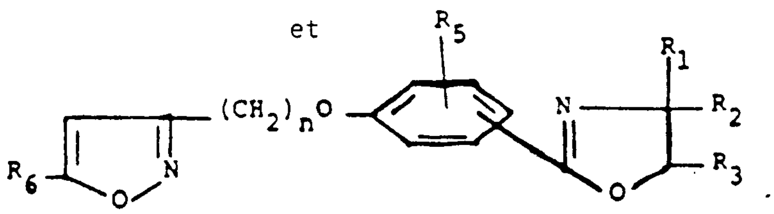

...II

dans lesquelles

R, $R_1$, $R_2$, $R_3$ et $R_4$ sont chacun un atome d'hydrogène ou un groupe alkyle en $C_1$-$C_3$ facultativement substitué par hydroxy, alcanoyloxy inférieur en $C_1$-$C_4$, alcoxy inférieur en $C_1$-$C_4$, chloro ou -N=Z, qui est un groupe amino, alcanoylamino inférieur en $C_1$-$C_4$, 1-pyrrolidinyle, 1-pipéridinyle ou 4-morpholinyle ; avec la condition que R soit autre que l'hydrogène ;

$R_5$ est un atome d'hydrogène ou d'halogène ou un groupe alkyle inférieur en $C_1$-$C_4$, nitro, alcoxy inférieur en $C_1$-$C_4$, alkylthio inférieur en $C_1$-$C_4$ ou trifluorométhyle ;

$R_6$ est un groupe alkyle en $C_1$-$C_3$ ;

X est O ou une liaison simple ; et

n est un entier de 3 à 9 ;

ou un de ses sels d'addition d'acides acceptables en pharmacie.

**2.** Un composé selon la revendication 1 : le 5-{7-[4-(4,5-dihydro-2-oxazolyl)phénoxy]heptyl}-3-méthylisoxazole ou un de ses sels d'addition d'acides acceptables en pharmacie.

**3.** Un composé selon la revendication 1 : le 5-{7-[2-chloro-4-(4,5-dihydro-2-oxazolyl)phénoxy]heptyl}-3-méthylixoxazole ou un de ses sels d'addition d'acides acceptables en pharmacie.

**4.** Un composé selon la revendication 1 : le 5-{5-[2-chloro-4-(4,5-dihydro-2-oxazolyl)phénoxy]pentyl}-3-méthylisoxazole ou un de ses sels d'addition d'acides acceptables en pharmacie.

**5.** Un composé de formule :

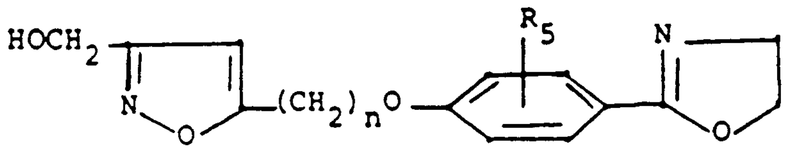

III

ou de formule

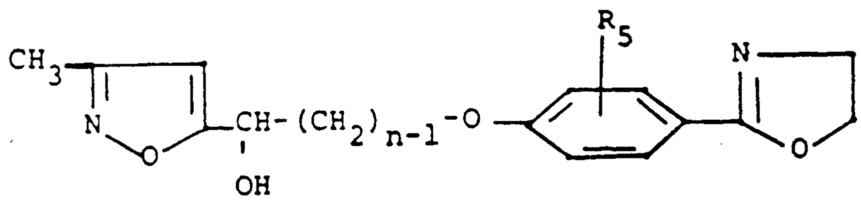

IV

dans lesquelles $R_5$ est un atome d'hydrogène ou d'halogène ou un groupe alkyle inférieur en $C_1$-$C_4$, nitro, alcoxy inférieur en $C_1$-$C_4$, alkylthio inférieur en $C_1$-$C_4$ ou trifluorométhyle et n est un entier de 3 à 9, ou un de ses sels d'addition d'acides acceptables en pharmacie.

**6.** Un procédé pour fabriquer un composé selon la revendication 1, qui comprend les étapes suivantes :

a. on fait réagir un composé de formule :

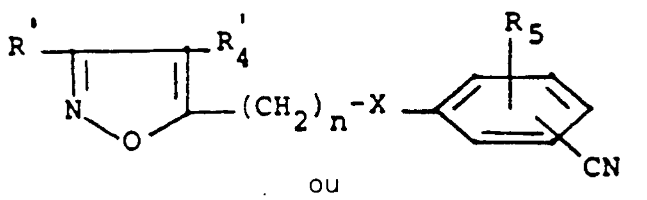

ou

...V

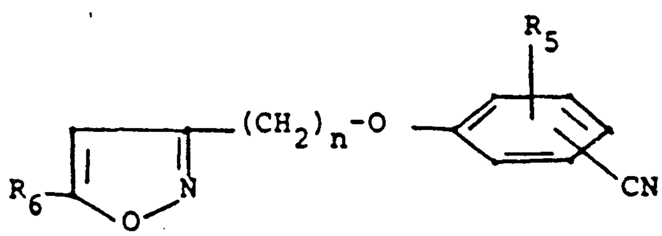

...VI

avec un alcanol inférieur en présence d'un acide fort et on chauffe l'imino-ester résultant avec un composé de formule :

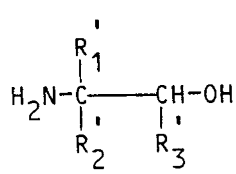

dans lesquelles R' est un groupe alkyle ou hydroxyalkyle en $C_1$-$C_3$ et $R'_1$, $R'_2$, $R'_3$ et $R'_4$ sont chacun un atome d'hydrogène ou un groupe alkyle ou hydroxyalkyle en $C_1$-$C_3$, pour préparer le composé de formule I ou II correspondant, ou

b. on fait réagir un composé de formule :

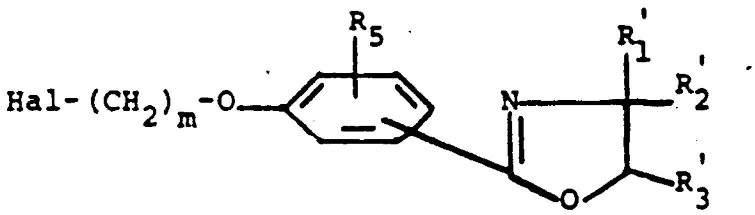

...VII

dans laquelle Hal est le brome ou l'iode et m = n-1, avec un dérivé de métal alcalin de 3-R'-4-$R'_4$-5-méthylisoxazole, dans lequel R' est un groupe alkyle ou hydroxyalkyle en $C_1$-$C_3$, $R'_1$, $R'_2$, $R'_3$ et $R'_4$ sont chacun un atome d'hydrogène ou un groupe alkyle ou hydroxyalkyle en $C_1$-$C_3$, de manière à préparer le composé de formule I correspondant dans lequel X est O, ou

c. on fait réagir un composé de formule :

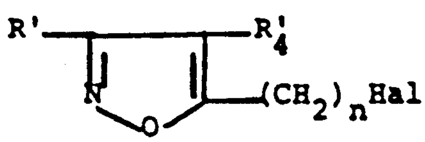

avec un sel de métal alcalin d'un composé de formule :

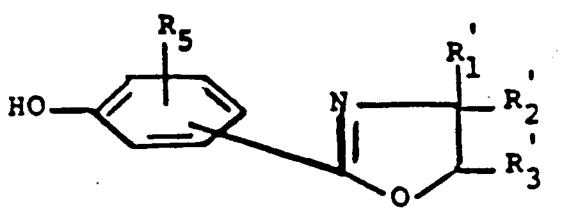

...IX

dans laquelle R' est un groupe alkyle ou hydroxyalkyle en $C_1$-$C_3$ ; $R'_1$, $R'_2$, $R'_3$ et $R'_4$ sont chacun un atome d'hydrogène ou un groupe alkyle ou hydroxyalkyle en $C_1$-$C_3$ de manière à préparer le composé de formule I correspondant dans lequel X est O,

si on le désire, on transforme un composé de formule I ou II ainsi obtenu dans lequel l'un au moins des restes R, $R_1$, $R_2$, $R_3$ et $R_4$ est un groupe hydroxyalkyle, de la manière suivante :

d. on estérifie ledit composé pour préparer un composé correspondant dans lequel l'un au moins desdits radicaux est un groupe alcanoyloxyalkyle inférieur,

e. on éthérifie ledit composé pour préparer le composé correspondant dans lequel l'un au moins desdits radicaux est un groupe alcoxyalkyle inférieur,

f. on soumet ledit composé à l'action d'un réactif capable de remplacer les groupes hydroxyles aliphatiques par du chlore pour préparer le composé correspondant dans lequel l'un au moins desdits radicaux est un groupe chloroalkyle,

g. on soumet un composé ainsi obtenu dans lequel l'un au moins des restes R, $R_1$, $R_2$, $R_3$ et $R_4$ est un groupe chloroalkyle, à la réaction avec l'ammoniac ou une amine de formule HN=Z, pour préparer le composé correspondant dans lequel le ou lesdits radicaux sont des groupes alkyl-N=Z, où N=Z est un groupe amino, alkylamino inférieur, di-(alkyl inférieur)amino, 1-pyrrolidinyle, 1-pipéridinyle ou 4-morpholinyle,

h. on fait réagir un composé ainsi obtenu dans lequel le ou lesdits radicaux sont des groupes aminoalkyles avec un halogénure d'alcanoyle inférieur ou l'anhydride correspondant pour pré parer le composé correspondant dans lequel le ou lesdits radicaux sont des groupes alcanoylaminoalkyles inférieurs et

si on le désire, on transforme une base libre ainsi obtenue en l'un de ses sels d'addition d'acides acceptables en pharmacie.

**7.** Un procédé pour préparer un composé selon la revendication 5, qui consiste à soumettre à une action enzymatique de fermentation convenable, par exemple en utilisant Aspergillus niger ($A_1$) ou Trichotecium roseum ($T_1$), un composé de formule I selon la revendication 1, dans laquelle R est $CH_3$, $R_1$, $R_2$, $R_3$ et $R_4$ sont H et X est O et le reste dihydro-oxazole est en position 4 du noyau phénylène et

si on le désire, on transforme une base libre ainsi obtenue en l'un de ses sels d'addition d'acides acceptables en pharmacie.

**8.** Un composé répondant à la formule IX :

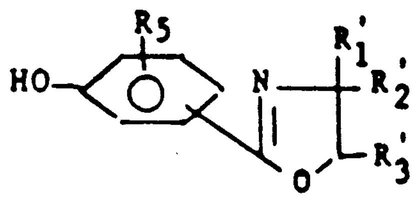

dans laquelle le groupe hydroxy est en position para par rapport au groupe oxazoline et/ou de formule VII :

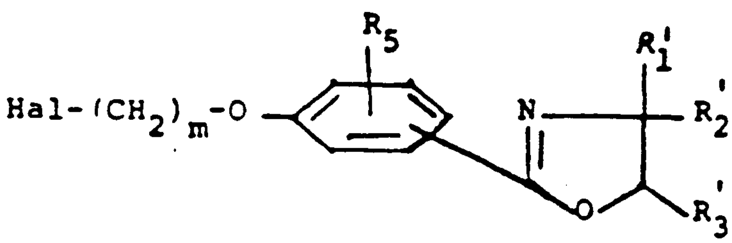

dans laquelle $R_1'$, $R_2'$ et $R_3'$ sont chacun un atome d'hydrogène ou un groupe alkyle ou hydroxyalkyle en $C_1$-$C_3$ ; $R_5$ est un atome d'hydrogène ou d'halogène ou un groupe alkyle inférieur en $C_1$-$C_4$, nitro, alcoxy inférieur en $C_1$-$C_4$, alkylthio inférieur en $C_1$-$C_4$ ou trifluorométhyle ; Hal est le brome ou l'iode ; et m est un entier de 2 à 8, à la condition que $R_1'$, $R_2'$, $R_3'$ et $R_5$ ne soient pas tous l'hydrogène.

**9.** Un procédé pour préparer un composé selon la revendication 8, qui consiste à cycliser un composé de formule :

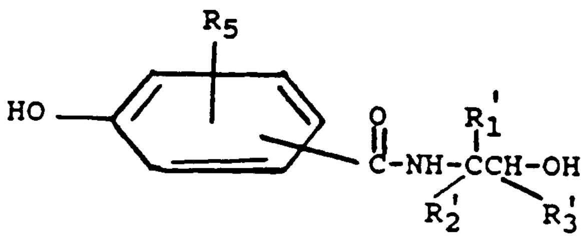

...XVII

pour préparer un composé de formule IX, si on le désire à éthérifier un composé de formule IX ainsi

obtenu par un dibromure d'alkylène pour obtenir un composé de formule VII dans laquelle Hal est le brome et si on le désire à remplacer le bromure par un iodure pour obtenir le composé de formule VII dans laquelle Hal est l'iode.

**10.** Une composition pour combattre les virus qui comprend une quantité efficace comme antiviral d'un composé selon l'une quelconque des revendications 1 à 5 en mélange avec un support ou diluant convenable.

**Revendications pour l'Etat contractant suivant : AT**

**1.** Un procédé pour préparer un composé de formule :

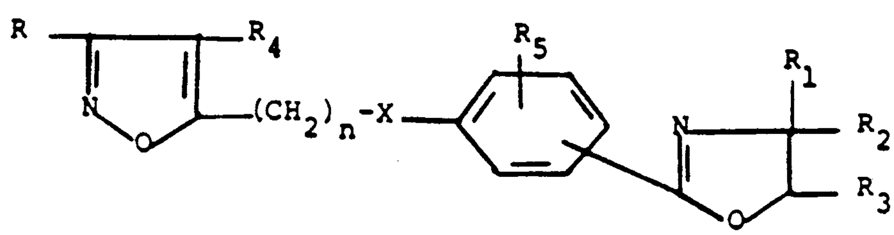

...I

ou de formule

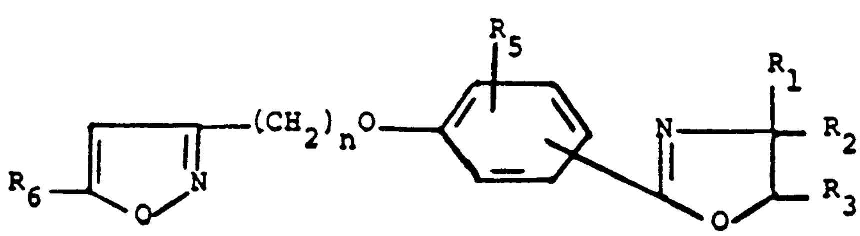

...II

dans lesquelles :

R, $R_1$, $R_2$, $R_3$ et $R_4$ sont chacun un atome d'hydrogène ou un groupe alkyle en $C_1$-$C_3$ facultativement substitué par hydroxy, alcanoyloxy inférieur en $C_1$-$C_4$, alcoxy inférieur en $C_1$-$C_4$, chloro ou -N = Z, qui est un groupe amino, alcanoylamino inférieur en $C_1$-$C_4$, 1-pyrrolidinyle, 1-pipéridinyle ou 4-morpholinyle ; avec la condition que R soit autre que l'hydrogène ;

$R_5$ est un atome d'hydrogène ou d'halogène ou un groupe alkyle inférieur en $C_1$-$C_4$, nitro, alcoxy inférieur en $C_1$-$C_4$, alkylthio inférieur en $C_1$-$C_4$ ou trifluorométhyle ;

$R_6$ est un groupe alkyle en $C_1$-$C_3$ ;

X est O ou une liaison simple ; et

n est un entier de 3 à 9 ;

ou un de ses sels d'addition d'acides acceptables en pharmacie, caractérisé en ce que :

a. on fait réagir un composé de formule :

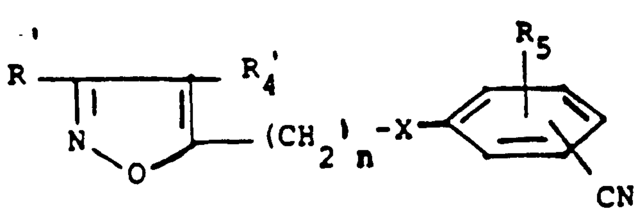

...V

ou

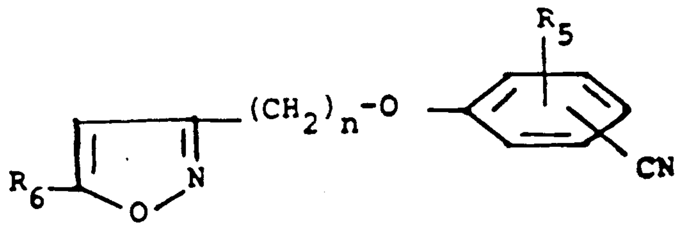

...VI

avec un alcanol inférieur en présence d'un acide fort et on chauffe l'imino-ester résultant avec un composé de formule :

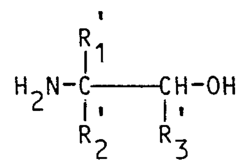

dans lesquelles R' est un groupe alkyle ou hydroxyalkyle en $C_1$-$C_3$ et $R'_1$ , $R'_2$ , $R'_3$ et $R'_4$ sont chacun un atome d'hydrogène ou un groupe alkyle ou hydroxyalkyle en $C_1$-$C_3$, pour préparer le composé de formule I ou II correspondant, ou

b. on fait réagir un composé de formule :

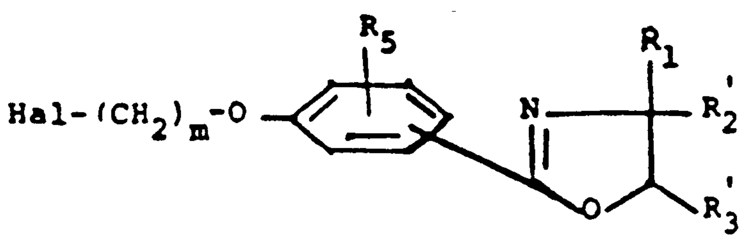

...VII

dans laquelle Hal est le brome ou l'iode et m = n-1, avec un dérivé de métal alcalin de 3-R'-4-$R'_4$ -5-méthylisoxazole, dans lequel R' est un groupe alkyle ou hydroxyalkyle en $C_1$-$C_3$, $R'_1$ , $R'_2$ , $R'_3$ et $R'_4$ sont chacun un atome d'hydrogène ou un groupe alkyle ou hydroxyalkyle en $C_1$-$C_3$, de manière à préparer le composé de formule I correspondant dans lequel X est O, ou

c. on fait réagir un composé de formule :

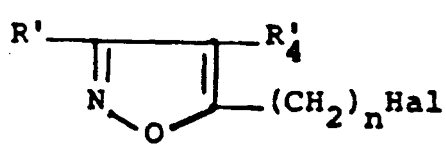

avec un sel de métal alcalin d'un composé de formule :

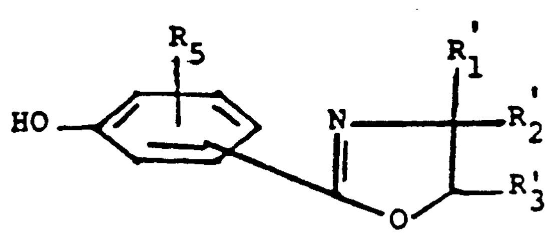

...IX

dans laquelle R' est un groupe alkyle ou hydroxyalkyle en $C_1$-$C_3$ ; $R_1'$, $R_2'$, $R_3'$ et $R_4'$ sont chacun un atome d'hydrogène ou un groupe alkyle ou hydroxyalkyle en $C_1$-$C_3$ de manière à préparer le composé de formule I correspondant dans lequel X est O,

si on le désire, on transforme un composé de formule I ou II ainsi obtenu dans lequel l'un au moins des restes R, $R_1$, $R_2$, $R_3$ et $R_4$ est un groupe hydroxyalkyle, de la manière suivante :

d. on estérifie ledit composé pour préparer un composé correspondant dans lequel l'un au moins desdits radicaux est un groupe alcanoyloxyalkyle inférieur,

e. on ethérifie ledit composé pour préparer le composé correspondant dans lequel l'un au moins desdits radicaux est un groupe alcoxyalkyle inférieur,

f. on soumet ledit composé à l'action d'un réactif capable de remplacer les groupes hydroxyles aliphatiques par du chlore pour préparer le composé correspondant dans lequel l'un au moins desdits radicaux est un groupe chloroalkyle,

g. on soumet un composé ainsi obtenu dans lequel l'un au moins des restes R, $R_1$, $R_2$, $R_3$ et $R_4$ est un groupe chloroalkyle, à la réaction avec l'ammoniac ou une amine de formule HN = Z, pour préparer le composé correspondant dans lequel le ou lesdits radicaux sont des groupes alkyl-N = Z, où N = Z est un groupe amino, alkylamino inférieur, di-(alkyl inférieur)amino, 1-pyrrolidinyle, 1-pipéridinyle ou 4-morpholinyle,

h. on fait réagir un composé ainsi obtenu dans lequel le ou lesdits radicaux sont des groupes aminoalkyles avec un halogénure d'alcanoyle inférieur ou l'anhydride correspondant pour préparer le composé correspondant dans lequel le ou lesdits radicaux sont des groupes alcanoylaminoalkyle inférieurs et

si on le désire, on transforme une base libre ainsi obtenue en l'un de ses sels d'addition d'acides acceptables en pharmacie.

**2.** Un procédé selon la revendication 1, dans lequel on prépare le 5-{7-[4-(4,5-dihydro-2-oxazolyl)-phénoxy]heptyl}-3-méthylisoxazole en faisant réagir le 5-[7-(4-cyanophénoxy)-heptyl]-3-méthylisoxazole avec un alcanol inférieur en présence d'un acide fort et on chauffe l'imino-ester résultant avec la 2-hydroxyéthylamine.

**3.** Un procédé selon la revendication 1, dans lequel on fait réagir un halogénure de 6-[4-(4,5-dihydro-2-oxazolyl)phénoxy]-hexyle avec un dérivé de métal alcalin du 3,5-diméthylisoxazole pour produire le 5-{7-[4-(4,5-dihydro-2-oxazolyl)-phénoxy]heptyl}-3-méthylisoxazole.

**4.** Un procédé selon la revendication 1, dans lequel on fait réagir un 5-(6-halogénohexyl)-3-méthylisoxazole avec un sel de métal alcalin du 2-(4-hydroxyphényl)-4,5-dihydrooxazole pour produire le 5-{7-[4-(4,5-dihydro-2-oxazolyl)phénoxy]heptyl}-3-méthylisoxazole.

5. Un procédé pour préparer un composé de formule :

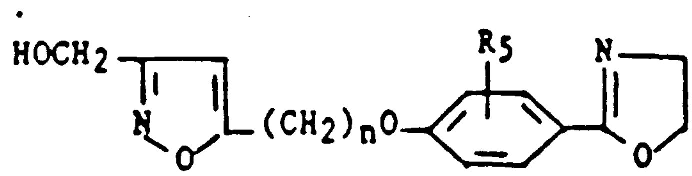

III

ou de formule

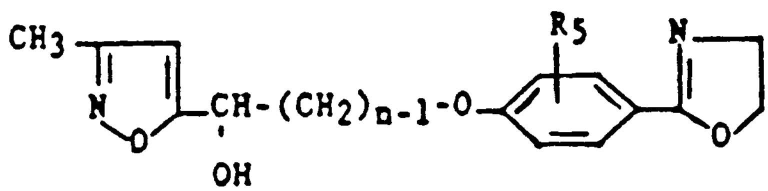

IV

dans lesquelles $R_5$ est un atome d'hydrogène ou d'halogène ou un groupe alkyle inférieur en $C_1$-$C_4$, nitro, alcoxy inférieur en $C_1$-$C_4$, alkylthio inférieur en $C_1$-$C_4$ ou trifluorométhyle et n est un entier de 3 à 9, ou un de ses sels d'addition d'acides acceptables en pharmacie, caractérisé en ce que l'on soumet à une action enzymatique de fermentation convenable un composé de formule I telle que définie à la revendication 1, dans laquelle R est $CH_3$, $R_1$, $R_2$, $R_3$ et $R_4$ sont H et X est O et le reste dihydro-oxazole est en position 4 du noyau phénylène et

si on le désire, on transforme une base libre ainsi obtenue en l'un de ses sels d'addition d'acides acceptables en pharmacie.

6. Un procédé selon la revendication 5, dans lequel $R_5$ est l'hydrogène et n est égal à 7, dans lequel ladite action enzymatique est produite en utilisant Aspergillus niger ($A_1$) ou Trichotecium roseum ($T_1$).

7. Un procédé pour préparer un composé de formule IX ou VII dans lesquelles $R'_1$ , $R'_2$ et $R'_3$ sont chacun un atome d'hydrogène ou un groupe alkyle ou hydroxyalkyle en $C_1$-$C_3$ ; $R_5$ est un atome d'hydrogène ou d'halogène ou un groupe alkyle inférieur en $C_1$-$C_4$, nitro, alcoxy inférieur en $C_1$-$C_4$, alkylthio inférieur en $C_1$-$C_4$ ou trifluorométhyle ; Hal est le brome ou l'iode ; et m est un entier de 2 à 8, caractérisé en ce que l'on cyclise un composé de formule :

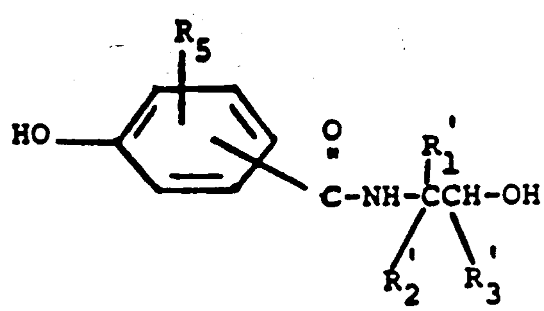

...XVII

pour préparer un composé de formule IX, si on le désire à éthérifier un composé de formule IX ainsi obtenu par un dibromure d'alkylène pour obtenir un composé de formule VII dans laquelle Hal est le brome et si on le désire à remplacer le bromure par un iodure pour obtenir le composé de formule VII dans laquelle Hal est l'iode.

8. Un procédé selon l'une quelconque des revendications 1, 5 et 7, dans lequel les composés de départ sont choisis de telle sorte que dans le composé de formule I produit, $R_1$ et $R_6$ soient chacun un groupe alkyle en $C_1$-$C_3$, $R_1$ et $R_2$ soient chacun l'hydrogène ou un groupe alkyle ou hydroxyalkyle en $C_1$-$C_3$, $R_3$, $R_4$ et $R_5$ soient chacun l'hydrogène, n soit un entier de 4 à 8 lorsque le groupe alkylène $(CH_2)_n$ est séparé par O du noyau phénylène ou un entier de 5 à 9 dans la formule I lorsque X est une liaison simple, et le reste dihydro-oxazole est fixé en position 4 du noyauphénylène.

9. Un procédé selon l'une quelconque des revendications 1, 5 et 7, dans lequel les composés de départ sont choisis de manière à exclure dans les composés de formule I produit les composés selon la revendication 8.

**Patentansprüche**

**Patentansprüche für folgende Vertragsstaaten : BE, CH, DE, FR, GB, IT, LI, NL, SE**

1. Verbindung der Formel

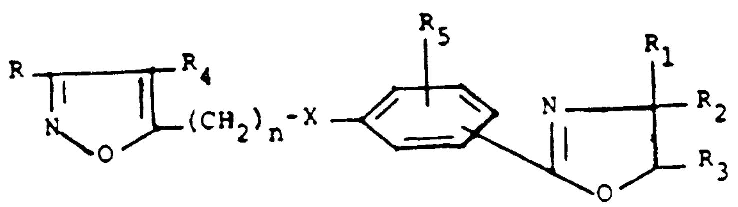

(I)

oder

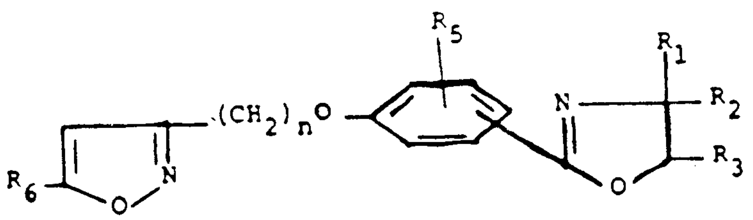

(II),

worin R, $R_1$, $R_2$, $R_3$ und $R_4$ jeweils Wasserstoff oder Alkyl mit 1 bis 3 Kohlenstoffatomen gegebenenfalls substituiert durch Hydroxy, nied.Alkanoyloxy mit 1 bis 4 Kohlenstoffatomen, nied.Alkoxy mit 1 bis 4 Kohlenstoffatomen, Chlor oder N=Z bedeuten, wobei N=Z Amino, nied.Alkanoylamino mit 1 bis 4 Kohlenstoffatomen, 1-Pyrrolidinyl, 1-Piperidinyl oder 4-Morpholinyl darstellt; mit der Maßgabe, daß R eine andere Bedeutung als Wasserstoff hat;

$R_5$ Wasserstoff, nied.Alkyl mit 1 bis 4 Kohlenstoffatomen, Halogen, Nitro, nied.Alkoxy mit 1 bis 4 Kohlenstoffatomen, nied.Alkylthio mit 1 bis 4 Kohlenstoffatomen oder Trifluormethyl bedeutet;

$R_6$ Alkyl mit 1 bis 3 Kohlenstoffatomen darstellt;

X die Bedeutung O hat oder eine Einfachbindung ist und

n eine ganze Zahl von 3 bis 9 ist,

oder ein pharmazeutisch annehmbares Säureadditionssalz hievon.

2. Verbindung nach Anspruch 1: 5-{7-[4-(4,5-Dihydro-2-oxazolyl)-phenoxy]-heptyl}-3-methylisoxazol oder ein pharmazeutisch annehmbares Säureadditionssalz hievon.

3. Verbindung nach Anspruch 1: 5-{7-[2-Chlor-4-(4,5-dihydro-2-oxazolyl)-phenoxy]-heptyl}-3-methylisoxazol oder ein pharmazeutisch annehmbares Säureadditionssalz hievon.

4. Verbindung nach Anspruch 1: 5-{5-[2-Chlor-4-(4,5-dihydro-2-oxazolyl)-phenoxy]-pentyl}-3-methylisoxazol oder ein pharmazeutisch annehmbares Säureadditionssalz hievon.

**5.** Verbindung der Formel

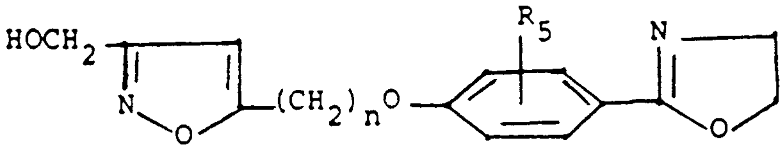

(III)

oder

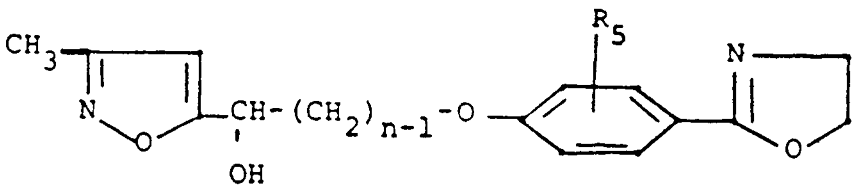

(IV),

worin $R_5$ Wasserstoff, nied.Alkyl mit 1 bis 4 Kohlenstoffatomen, Halogen, Nitro, nied.Alkoxy mit 1 bis 4 Kohlenstoffatomen, nied.Alkylthio mit 1 bis 4 Kohlenstoffatomen oder Trifluormethyl bedeutet und n eine ganze Zahl von 3 bis 9 ist, oder ein pharmazeutisch annehmbares Säureadditionssalz hievon.

**6.** Verfahren zum Herstellen einer Verbindung nach Anspruch 1, das umfaßt:
a. das Umsetzen einer Verbindung der Formel

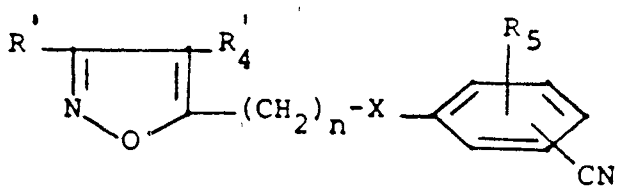

(V)

oder

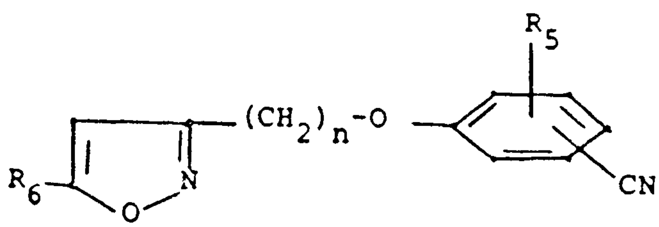

(VI)

mit einem niederen Alkanol in Anwesenheit einer starken Säure und Erhitzen des erhaltenen Iminoesters mit einer Verbindung der Formel

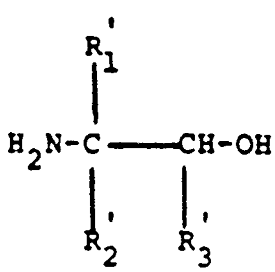

,

worin R' Alkyl oder Hydroxyalkyl mit 1 bis 3 Kohlenstoffatomen bedeutet und $R_1'$, $R_2'$, $R_3'$ und $R_4'$ jeweils Wasserstoff, Alkyl oder Hydroxyalkyl mit 1 bis 3 Kohlenstoffatomen sind, um die entsprechende Verbindung der Formel (I) oder (II) herzustellen, oder

b. das Umsetzen einer Verbindung der Formel

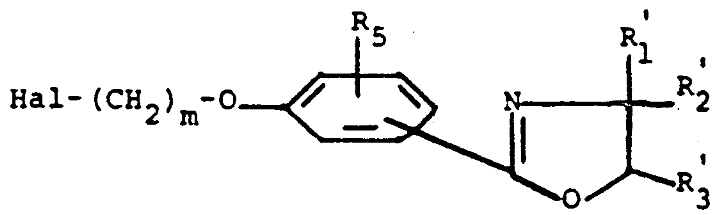

(VII),

worin Hal Brom oder Jod bedeutet und m = n-1, mit einem Alkalimetallderivat von 3-R'-4-$R_4$'-5-Methylisoxazol, wobei R' Alkyl oder Hydroxyalkyl mit 1 bis 3 Kohlenstoffatomen bedeutet und $R_1'$, $R_2'$, $R_3'$ und $R_4'$ jeweils Wasserstoff, Alkyl oder Hydroxyalkyl mit 1 bis 3 Kohlenstoffatomen sind, um die entsprechende Verbindung der Formel (I) herzustellen, worin X die Bedeutung O hat, oder

c. das Umsetzen einer Verbindung der Formel

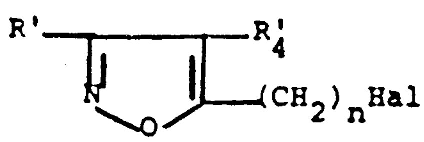

mit einem Alkalimetallsalz einer Verbindung der Formel

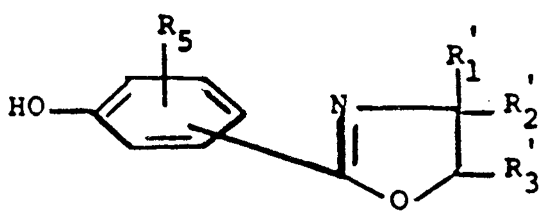

(IX),

worin R' Alkyl oder Hydroxyalkyl mit 1 bis 3 Kohlenstoffatomen bedeutet und $R_1'$, $R_2'$, $R_3'$ und $R_4'$ jeweils Wasserstoff, Alkyl oder Hydroxyalkyl mit 1 bis 3 Kohlenstoffatomen sind, um die entsprechende Verbindung der Formel (I) herzustellen, worin X die Bedeutung O hat,

und, wenn gewünscht, das Überführen einer erhaltenen Verbindung der Formel (I) oder (II), worin einer oder mehrere der Reste R, $R_1$, $R_2$, $R_3$ oder $R_4$ Hydroxyalkyl sind, wie folgt:

d. Verestern der Verbindung zum Herstellen der entsprechenden Verbindung, worin einer oder mehrere dieser Reste nied.Alkanoyloxyalkyl sind,

e. Veräthern der Verbindung zum Herstellen der entsprechenden Verbindung, worin einer oder mehrere dieser Reste nied.Alkoxyalkyl sind,

f. Aussetzen der Verbindung der Einwirkung eines Reagens, das imstande ist, aliphatische Hydroxylgruppen durch Chlor zu ersetzen, um die entsprechende Verbindung herzustellen, worin einer oder mehrere dieser Reste Chloralkyl sind,

g. Unterwerfen einer so erhaltenen Verbindung, worin einer oder mehrere der Reste R, $R_1$, $R_2$, $R_3$ oder $R_4$ Chloralkyl sind, der Reaktion mit Ammoniak oder einem Amin, HN=Z, um die entsprechende Verbindung herzustellen, worin dieser Rest oder diese Reste Alkyl-N=Z sind, wobei N=Z Amino, nied.Alkylamino, Di-nied.alkylamino, 1-Pyrrolidinyl, 1-Piperidinyl oder 4-Morpholinyl bedeutet,

h. Umsetzen einer so erhaltenen Verbindung, worin einer oder mehrere dieser Reste Aminoalkyl sind, mit einem nied.Alkanoylhalogenid oder Anhydrid, um die entsprechende Verbindung herzustellen, worin einer oder mehrere dieser Reste nied.Alkanoylaminoalkyl sind, und,

wenn gewünscht, Überführen einer erhaltenen freien Base in ein pharmazeutisch annehmbares Säureadditionssalz hievon.

**7.** Verfahren zum Herstellen einer Verbindung nach Anspruch 5, das das Aussetzen einer Verbindung der Formel (I) nach Anspruch 1, worin R $CH_3$ bedeutet, $R_1$, $R_2$, $R_3$ und $R_4$ die Bedeutung H haben, X O darstellt und die Dihydrooxazolgruppe in Stellung 4 des Phenylenringes ist, einer geeigneten fermentativen enzymatischen Einwirkung, beispielsweise unter Verwendung von Aspergillus niger ($A_1$) oder Trichotecium roseum ($T_1$), und,

wenn gewünscht, Überführen einer erhaltenen freien Base in ein pharmazeutisch annehmbares Salz hievon umfaßt.

**8.** Verbindung der Formel (IX)

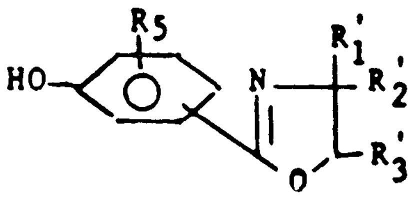

,

worin die Hydroxygruppe in p-Stellung zur Oxazolingruppe ist, und/oder (VII)

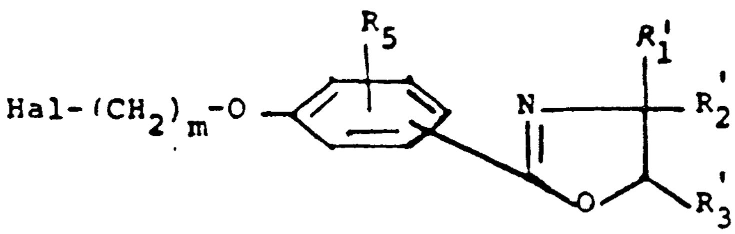

,

worin $R_1'$, $R_2'$ und $R_3'$ jeweils Wasserstoff, Alkyl oder Hydroxyalkyl mit 1 bis 3 Kohlenstoffatomen bedeuten, $R_5$ Wasserstoff, nied.Alkyl mit 1 bis 4 Kohlenstoffatomen, Halogen, Nitro, nied.Alkoxy mit 1 bis 4 Kohlenstoffatomen, nied.Alkylthio mit 1 bis 4 Kohlenstoffatomen oder Trifluormethyl darstellt, Hal Brom oder Jod ist und m eine ganze Zahl von 2 bis 8 ist, mit der Maßgabe, daß nicht alle Reste $R_1'$, $R_2'$, $R_3'$ und $R_5$ Wasserstoff sind.

**9.** Verfahren zum Herstellen einer Verbindung nach Anspruch 8, das das Cylisieren einer Verbindung der Formel

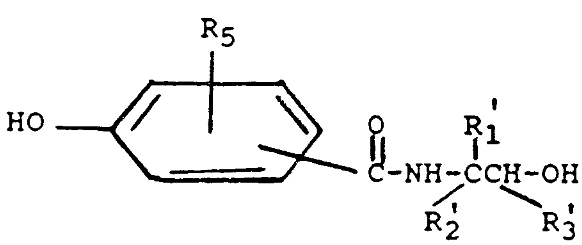

(XVII)

zum Herstellen einer Verbindung der Formel (IX), wenn gewünscht, Veräthern einer erhaltenen

Verbindung der Formel (IX) mit einem Alkylendibromid zum Erhalten einer Verbindung der Formel (VII), worin Hal Brom bedeutet, und, wenn gewünscht, Ersetzen des Bromids durch ein Jodid zum Erhalten der Verbindung der Formel (VII), worin Hal Jod ist, umfaßt.

**10.** Zusammensetzung zum Bekämpfen von Viren, die eine antiviral wirksame Menge einer Verbindung nach einem der Ansprüche 1 bis 5 in Mischung mit einem geeigneten Träger oder Verdünnungsmittel umfaßt.

**Patentansprüche für folgenden Vertragsstaat : AT**

**1.** Verfahren zum Herstellen einer Verbindung der Formel

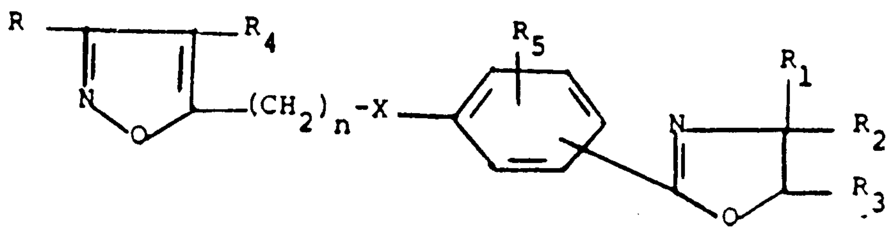
(I)

oder

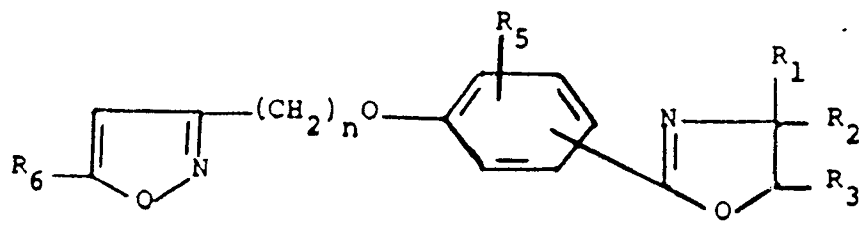
(II),

worin R, $R_1$, $R_2$, $R_3$ und $R_4$ jeweils Wasserstoff oder Alkyl mit 1 bis 3 Kohlenstoffatomen gegebenenfalls substituiert durch Hydroxy, nied.Alkanoyloxy, nied.Alkoxy, Chlor oder N = Z bedeuten, wobei N = Z Amino, nied.Alkanoylamino, 1-Pyrrolidinyl, 1-Piperidinyl oder 4-Morpholinyl darstellt; mit der Maßgabe, daß R eine andere Bedeutung als Wasserstoff hat;

$R_5$ Wasserstoff, nied.Alkyl mit 1 bis 4 Kohlenstoffatomen, Halogen, Nitro, nied.Alkoxy mit 1 bis 4 Kohlenstoffatomen, nied.Alkylthio mit 1 bis 4 Kohlenstoffatomen oder Trifluormethyl bedeutet;

$R_6$ Alkyl mit 1 bis 3 Kohlenstoffatomen darstellt;

X die Bedeutung O hat oder eine Einfachbindung ist und

n eine ganze Zahl von 3 bis 9 ist,

oder eines pharmazeutisch annehmbaren Säureadditionssalzes hievon, gekennzeichnet durch

a. Umsetzen einer Verbindung der Formel

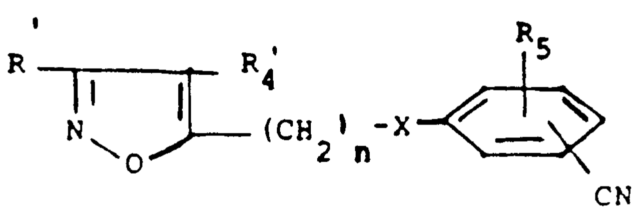
(V)

oder

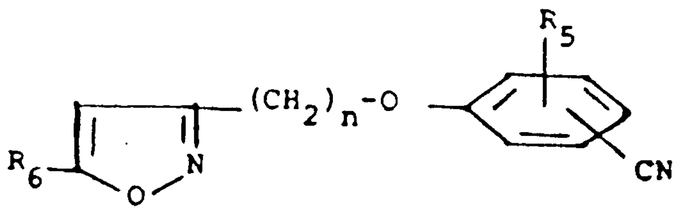

(VI)

mit einem niederen Alkanol in Anwesenheit einer starken Säure und Erhitzen des erhaltenen Iminoesters mit einer Verbindung der Formel

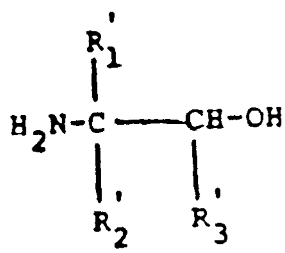

,

worin R' Alkyl oder Hydroxyalkyl mit 1 bis 3 Kohlenstoffatomen bedeutet und $R_1$', $R_2$', $R_3$' und $R_4$' jeweils Wasserstoff, Alkyl oder Hydroxyalkyl mit 1 bis 3 Kohlenstoffatomen sind, um die entsprechende Verbindung der Formel (I) oder (II) herzustellen, oder

b. Umsetzen einer Verbindung der Formel

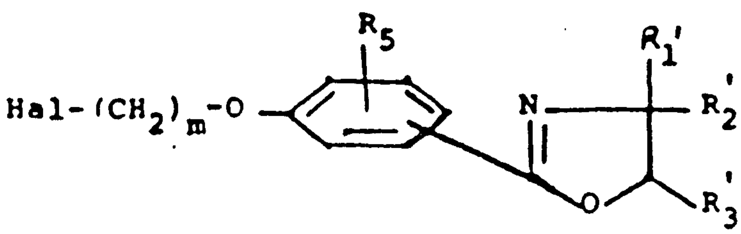

(VII),

worin Hal Brom oder Jod bedeutet und m = n-1, mit einem Alkalimetallderivat von 3-R'-4-$R_4$'-5-Methylisoxazol, wobei R' Alkyl oder Hydroxyalkyl mit 1 bis 3 Kohlenstoffatomen bedeutet, und $R_1$', $R_2$' $R_3$', und $R_4$' jeweils Wasserstoff, Alkyl oder Hydroxyalkyl mit 1 bis 3 Kohlenstoffatomen sind, um die entsprechende Verbindung der Formel (I) herzustellen, worin X die Bedeutung O hat, oder

c. Umsetzen einer Verbindung der Formel

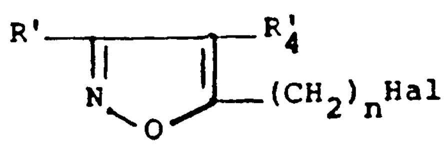

mit einem Alkalimetallsalz einer Verbindung der Formel

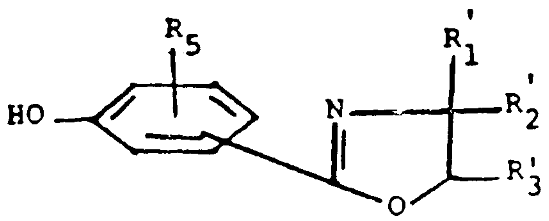

(IX),

worin R' Alkyl oder Hydroxyalkyl mit 1 bis 3 Kohlenstoffatomen bedeutet und $R_1$', $R_2$', $R_3$' und $R_4$' jeweils Wasserstoff, Alkyl oder Hydroxyalkyl mit 1 bis 3 Kohlenstoffatomen sind, um die entsprechende Verbindung der Formel (I) herzustellen, worin X die Bedeutung O hat,

und, wenn gewünscht, Überführen einer erhaltenen Verbindung der Formel (I) oder (II), worin einer oder mehrere der Reste R, $R_1$, $R_2$, $R_3$ oder $R_4$ Hydroxyalkyl sind, wie folgt:

d. Verestern der Verbindung zum Herstellen der entsprechenden Verbindung, worin einer oder mehrere dieser Reste nied.Alkanoyloxyalkyl sind,

e. Veräthern der Verbindung zum Herstellen der entsprechenden Verbindung, worin einer oder mehrere dieser Reste nied.Alkoxyalkyl sind,

f. Aussetzen der Verbindung der Einwirkung eines Reagens, das imstande ist, aliphatische Hydroxylgruppen durch Chlor zu ersetzen, um die entsprechende Verbindung herzustellen, worin einer oder mehrere dieser Reste Chloralkyl sind,

g. Unterwerfen einer so erhaltenen Verbindung, worin einer oder mehrere von R, $R_1$, $R_2$, $R_3$ oder $R_4$ Chloralkyl sind, der Reaktion mit Ammoniak oder einem Amin, HN=Z, um die entsprechende Verbindung herzustellen, worin dieser Rest oder diese Reste Alkyl-N=Z sind, wobei N=Z Amino, nied.Alkylamino, Di-nied.alkylamino, 1-Pyrrolidinyl, 1-Piperidinyl oder 4-Morpholinyl bedeutet,

h. Umsetzen einer so erhaltenen Verbindung, worin einer oder mehrere dieser Reste Aminoalkyl sind, mit einem nied.Alkanoylhalogenid oder Anhydrid, um die entsprechende Verbindung herzustellen, worin einer oder mehrere dieser Reste nied.Alkanoylaminoalkyl sind, und,

wenn gewünscht, Überführen einer erhaltenen freien Base in ein pharmazeutisch annehmbares Säureadditionssalz hievon.

2. Verfahren nach Anspruch 1, worin 5-{7-[4-(4,5-Dihydro-2-oxazolyl)-phenoxy]-heptyl}-3-methylisoxazol hergestellt wird durch Umsetzen von 5-[7-(4-Cyanophenoxy)-heptyl]-3-methylisoxazol mit einem niederen Alkanol in Anwesenheit einer starken Säure und Erhitzen des erhaltenen Iminoesters mit 2-Hydroxyäthylamin.

3. Verfahren nach Anspruch 1, worin 6-[4-(4,5-Dihydro-2-oxazolyl)-phenoxy]-hexylhalogenid mit einem Alkalimetallderivat von 3,5-Dimethylisoxazol zum Herstellen von 5-{7-[4-(4,5-Dihydro-2-oxazolyl)-phenoxy]-heptyl}-3-methylisoxazol umgesetzt wird.

4. Verfahren nach Anspruch 1, worin 5-(6-Halogenhexyl)-3-methylisoxazol mit einem Alkalimetallsalz von 2-(4-Hydroxyphenyl)-4,5-dihydrooxazol zum Herstellen von 5-{7-[4-(4,5-Dihydro-2-oxazolyl)-phenoxy]-heptyl}-3-methylisoxazol umgesetzt wird.

5. Verfahren zum Herstellen einer Verbindung der Formel

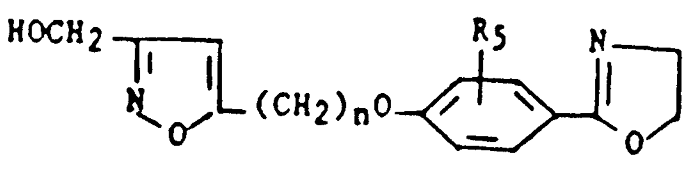

(III)

oder

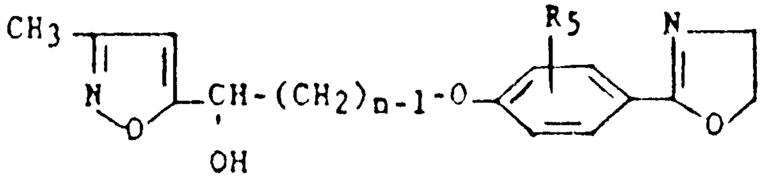

(IV),

worin $R_5$ Wasserstoff, nied.Alkyl mit 1 bis 4 Kohlenstoffatomen, Halogen, Nitro, nied.Alkoxy mit 1 bis 4 Kohlenstoffatomen, nied.Alkylthio mit 1 bis 4 Kohlenstoffatomen oder Trifluormethyl bedeutet und n eine ganze Zahl von 3 bis 9 ist, oder eines pharmazeutisch annehmbaren Säureadditionssalzes hievon, dadurch gekennzeichnet, daß eine Verbindung der Formel (I), wie in Anspruch 1 definiert, worin R $CH_3$ bedeutet, $R_1$, $R_2$, $R_3$ und $R_4$ die Bedeutung H haben, X O darstellt und die Dihydrooxazolgruppe in Stellung 4 des Phenylenringes ist, einer geeigneten fermentativen enzymatischen Einwirkung unterworfen und, wenn gewünscht, eine erhaltene freie Base in ein pharmazeutisch annehmbares Säureadditionssalz hievon übergeführt wird.

**6.** Verfahren nach Anspruch 5, daß eine Verbindung, worin $R_5$ Wasserstoff bedeutet und n 7 ist, unter Verwendung von Aspergillus niger ($A_1$) oder Trichotecium roseum ($T_1$) hergestellt wird.

**7.** Verfahren zum Herstellen einer Verbindung der Formel (IX) oder (VII), worin $R_1'$, $R_2'$ und $R_3'$ jeweils Wasserstoff, Alkyl oder Hydroxyalkyl mit 1 bis 3 Kohlenstoffatomen bedeuten, $R_5$ Wasserstoff, nied.Alkyl mit 1 bis 4 Kohlensfoffatomen, Halogen, Nitro, nied.Alkoxy mit 1 bis 4 Kohlenstoffatomen, nied.Alkylthio mit 1 bis 4 Kohlenstoffatomen oder Trifluormethyl darstellt, Hal Brom oder Jod ist und m eine ganze Zahl von 2 bis 8 ist, dadurch gekennzeichnet, daß eine Verbindung der Formel

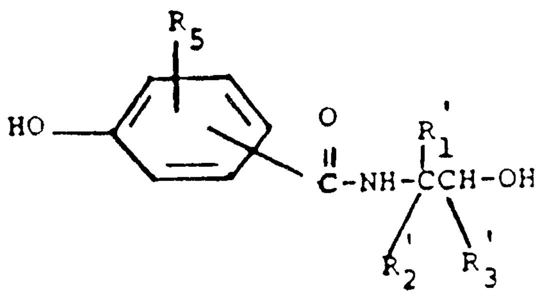

(XVII)

zum Herstellen einer Verbindung der Formel (IX) cyclisiert wird, wenn gewünscht, eine erhaltene Verbindung der Formel (IX) mit einem Alkylendibromid zum Erhalten einer Verbindung der Formel (VII), worin Hal Brom bedeutet, veräthert wird und, wenn gewünscht, das Bromid durch ein Jodid ersetzt wird, um die Verbindung der Formel (VII) zu erhalten, worin Hal Jod ist.

**8.** Verfahren nach einem der Ansprüche 1, 5 und 7, in dem die Ausgangsverbindungen so gewählt werden, daß in der gebildeten Verbindung der Formel (I) R und $R_6$ jeweils Alkyl mit 1 bis 3 Kohlenstoffatomen bedeuten, $R_1$ und $R_2$ jeweils Wasserstoff, Alkyl oder Hydroxyalkyl mit 1 bis 3 Kohlenstoffatomen darstellen, $R_3$, $R_4$ und $R_5$ jeweils Wasserstoff sind, n eine ganze Zahl von 4 bis 8 ist, wenn es vom Phenylenring durch O getrennt ist, oder eine ganze Zahl von 5 bis 9 in Formel (I) ist, wenn X eine Einfachbindung darstellt, und die Dihydrooxazolgruppe an die Stellung 4 des Phenylenringes gebunden ist.

**9.** Verfahren nach einem der Ansprüche 1, 5 und 7, in dem die Ausgangsverbindungen so gewählt werden, daß in der gebildeten Verbindung der Formel (I) eine Verbindung nach Anspruch 8 ausgeschlossen ist.